# EUROPEAN PATENT APPLICATION

(11) **EP 2 255 780 A1**
(43) Date of publication of application: **01.12.2010**
(21) Application number: 09718857.7
(22) Date of filing: 12.03.2009
(51) Int. Cl.: A61K 8/44, A61Q 19/00

(54) **PARAKERATOSIS INHIBITOR, PORE SHRINKING AGENT OR AGENT FOR PREVENTING OR IMPROVING ROUGH SKIN, AND COMPOSITION FOR EXTERNAL USE ON THE SKIN CONTAINING THE SAME**

(30) Priority: 12.03.2008 JP 2008063352; 28.03.2008 JP 2008087497
(71) Applicant: Shiseido Company, Ltd., Chuo-ku Tokyo 104-8010 (JP)
(72) Inventor: IIDA, Toshii, Yokohama-shi Kanagawa 224-8558 (JP); KANEKO, Maki, Yokohama-shi Kanagawa 224-8558 (JP); KAMINUMA, Mikiko, Yokohama-shi Kanagawa 224-8558 (JP); SUETSUGU, Masaru, Yokohama-shi Kanagawa 224-8558 (JP)
(74) Representative: Henkel, Feiler & Hänzel
(86) International application number: PCT/JP2009/054803
(87) International publication number: WO 2009/113635

(57) **Abstract**

The present invention provides a parakeratosis inhibitor, a pore-shrinking agent, and a rough skin inhibiting or ameliorating agent comprising, as an active ingredient, a compound that has excellent effects and high safety without safety problems such as sensory irritation, and provides an external composition for skin containing the same. The compound is selected from the glutamic acid derivatives represented by formula (1) or (2) and the salts thereof: 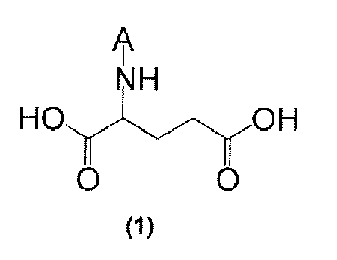 wherein A represents a carbamoyl group, a benzyloxycarbonyl group, an alkyl group having 1 to 3 carbon atoms, an allyl group, or an amidino group; 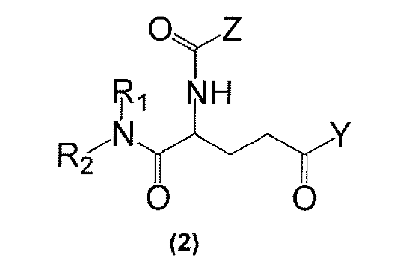 wherein Z represents an alkyl group having 1 to 4 carbon atoms, an alkenyl group having 2 to 4 carbon atoms, etc.; Y represents OH, an alkyloxy group having 1 to 3 carbon atoms, an allyloxy group, or NR₃R₄; R₁ to R₄ each independently represents H, an alkyl group having 1 to 3 carbon atoms, an allyl group, a cycloalkyl group having 3 to 7 carbon atoms, a cycloalkenyl group having 3 to 7 carbon atoms, a phenyl group, or a benzyl group, or R₁ and R₂, or R₃ and R₄, independently form a heterocycle.

## Description

### RELATED APPLICATIONS

This application claims the priorities of Japanese Patent Application No. 2008-63352 filed on March 12, 2008 and Japanese Patent Application No. 2008-87497 filed on March 28, 2008, which are incorporated herein by reference.

### FIELD OF THE INVENTION

The present invention relates to a parakeratosis inhibitor for inhibiting parakeratosis caused by sebum; a pore-shrinking agent for inhibiting parakeratosis caused by the irritant components in the sebum around the pore, maintaining skin around the pore in normal condition, and suppressing a funnel-shaped structure of the pore from becoming conspicuous; a rough skin inhibiting or ameliorating agent for inhibiting or ameliorating rough skin caused by unsaturated fatty acids; and an external composition for skin having these effects.

### BACKGROUND OF THE INVENTION

Today people, especially young women, are more concerned with conspicuous pores, and an external preparation for skin that ameliorates such conspicuous pores has been in demand. However, as the mechanism by which conspicuousness of pores grows has not been fully elucidated, it has been common to use an astringent lotion or remove keratin plug. Alternatively, a foundation is often used to improve the appearance. However, for example, an astringent lotion is intended to tighten the skin by temporarily lowering the skin surface temperature with an alcohol or coagulating proteins with an organic acid and the like. Thus, it put much stress on the skin and could not provide a fundamental solution to conspicuous pores or could not achieve sufficient effect.

Although there are reports that glycolic acid or ascorbic acid derivatives have pore-shrinking effects (for example, see Non-Patent Literature 1), there are still many unknowns, such as the mechanism of action and the extent of the effects.

Keratin plug removal is a method of physically removing a keratin plug which clogs the pore. In such a method, as the physical force may damage the skin, side effects to the skin have sometimes been serious problems. In addition, the effect of this method is not always satisfactory since the effect is so temporary that keratin plug is readily regenerated, and the removal of keratin plug may result in adversely expanding the pore.

In the normal skin, nuclei are lost in the terminal differentiation process from granular layer to stratum corneum, which is referred to as "denucleation". If this "denucleation" does not occur owing to any cause, epidermal keratinocytes in a state of having nuclei are formed. Such state is referred to as "parakeratosis".
The present inventors studied the mechanism of conspicuous pores. As a result, it has been revealed that the funnel-shaped depression surrounding a follicle is recognized as a pore, the pore becomes conspicuous when this area is enlarged, and furthermore the stratum corneum of this funnel-shaped area is in a state of parakeratosis (i.e., nuclei that should have disappeared still remain). Thus, it has been found that, if a parakeratosis inhibitor acts on the pores to inhibit parakeratosis of the stratum corneum of funnel-shaped depression surrounding a follicle, it reduces the area recognized as pores, and therefore, acts as a pore-shrinking agent. Moreover, it has been also reveled that people having conspicuous pores have a large amount of sebum, particularly a high ratio of unsaturated fatty acid, and that this unsaturated fatty acid causes parakeratosis. Thus, it has been found that a unsaturated fatty acid in sebum have potential to cause conspicuous pores. (see Non-patent Literatures 2 and 3).

As mentioned above, by the present inventors, it was made clear that parakeratosis caused by sebum contributes to the mechanism by which pores become conspicuous, and that therefore the amelioration of parakeratosis in the pores reduces the area recognized as pores, resulting in abating conspicuous pores. Based on the above-mentioned findings, the present inventors found a novel parakeratosis inhibitor and a novel pore-shrinking agent, filed a patent application of the same as an initial report (see Patent Literature 1). Furthermore, the present inventors found a novel preparation for parakeratosis, a novel pore-shrinking agent, and a novel rough skin inhibiting or ameliorating agent which were more excellent in the effects (see Patent Literatures 2 and 3).

γ-Aminobutyric acid (GABA) has been known to have effects of inhibiting parakeratosis and shrinking pores (see Patent Literature 4). However, there has been demand for a widely-usable parakeratosis inhibitor and pore-shrinking agent which would be higher in safety and pose no problem such as limit on the amount incorporated.
Patent Literature 2 describes, as glutamic acid derivatives having the effects of the present invention, benzoyl-L-glutamic acid, benzenesulfonyl-L-glutamic acid, acetyl-L-glutamic acid, etc.. However, the development of a compound which would be more excellent in the effects has been desired.

On the other hand, it has been reported that an inhibitor for nitric oxide synthetase (NOS) was incorporated in an external preparation for skin to inhibit skin pigmentation and was applied in the skin disease due to inflammation or skin dryness. In this context, N-amidinoglutamic acid was disclosed as an example of NOS inhibitors (see Patent Literatures 5 and 6). However, it has not been reported yet that NOS inhibitors or N-amidinoglutamic acids had an effect of inhibiting parakeratosis or abating conspicuous pores.

Patent Literature 1: Japanese Unexamined Patent Publication No. 2004-2289
Patent Literature 2: Japanese Unexamined Patent Publication No. 2005-179342
Patent Literature 3: Japanese Unexamined Patent Publication No. 2005-179343
Patent Literature 4: Japanese Unexamined Patent Publication No. 2003-342195
Patent Literature 5: PCT International Publication No. WO02/069910
Patent Literature 6: German Patent Publication No. 10111049
Non-patent Literature 1: Yazawa et al., Fragrance Journal, 2002, vol. 30, No. 2, p. 54 to 58.
Non-patent Literature 2: Iida et al., Program and Minutes of the 102nd Japanese Dermatological Association Convention, 2003, 103, p. 846.
Non-patent Literature 3: Katsuta et al., Cosmetics & Toiletries magazine, 2004, vol. 119, No. 10, p.59 to 64.

### DISCLOSURE OF THE INVENTION

### PROBLEM TO BE SOLVED BY THE INVENTION

The present invention was made in view of the above-described problems, and an object thereof is to provide a parakeratosis inhibitor, a pore-shrinking agent, a rough skin inhibiting or ameliorating agent, and an external composition for skin that have excellent effects of inhibiting parakeratosis, shrinking pores, and inhibiting or ameliorating rough skin and are high in safety without safety problems such as sensory irritation.

### MEANS TO SOLVE THE PROBLEM

To solve the above-described problems, the present inventors have diligently studied to search a compound which had excellent effects of inhibiting parakeratosis, shrinking pores, and inhibiting or ameliorating rough skin, were high in safety without safety problems such as sensory irritation, and were easy to be formulated. As a result, it was found that specific glutamic acid derivatives and salts thereof have excellent effects to solve the above-mentioned problems, thus leading to completion of the present invention.

That is, the present invention provides a parakeratosis inhibitor or pore-shrinking agent comprising, as an active ingredient, one or more compounds selected from the group consisting of glutamic acid derivatives represented by the following formula (1) or (2) and the salts thereof.

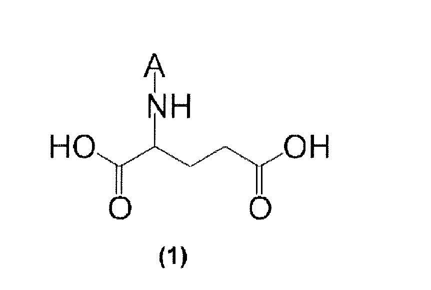

In the formula (1), A represents a carbamoyl group, a benzyloxycarbonyl group, an alkyl group having 1 to 3 carbon atoms, an allyl group, or an amidino group.

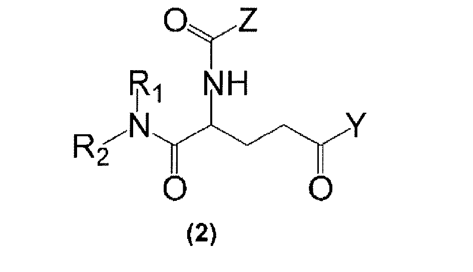

In the formula (2), Z represents an alkyl group having 1 to 4 carbon atoms, an alkenyl group having 2 to 4 carbon atoms, a benzyloxy group, or a group represented by the following formula (3) or (4);
Y represents a hydroxyl group, an alkyloxy group having 1 to 3 carbon atoms, an allyloxy group, or NR₃R₄; with the proviso that Y is not NR₃R₄ when Z is a group represented by the formula (4); and
R₁ to R₄ each independently represents a hydrogen atom, an alkyl group having 1 to 3 carbon atoms, an allyl group, a cycloalkyl group having 3 to 7 carbon atoms, a cycloalkenyl group having 3 to 7 carbon atoms, a phenyl group, or a benzyl group, or R₁ and R₂, and/or R₃ and R₄, independently form a heterocycle having 6 carbon atoms or less in total together with the nitrogen atom to which they are bonded, wherein the heterocycle may include an oxygen atom,
in R₁ to R₄, the alkyl group having 1 to 3 carbon atoms, the allyl group, the cycloalkyl group having 3 to 7 carbon atoms, the cycloalkenyl group having 3 to 7 carbon atoms, the phenyl group, the benzyl group, or the heterocycle may have, as a substituent, a hydroxyl group, an alkyloxy group having 1 to 3 carbon atoms, or an allyloxy group, and
in R₁ to R₄, the cycloalkyl group having 3 to 7 carbon atoms, the cycloalkenyl group having 3 to 7 carbon atoms, the phenyl group, the benzyl group, or the heterocycle may have, as a substituent, an alkyl group having 1 to 3 carbon atoms.

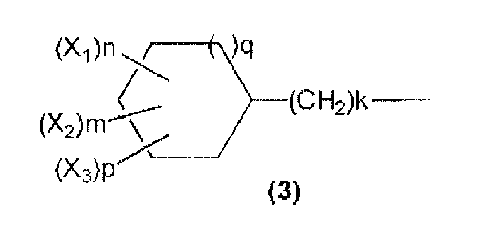

In the formula (3), X₁, X₂, and X₃ each independently represents an alkyl group having 1 to 4 carbon atoms, an alkenyl group having 1 to 4 carbon atoms, an alkyloxy group having 1 to 4 carbon atoms, an alkenyloxy group having 2 to 4 carbon atoms, a hydroxyl group, an amino group, an alkylamino group having 1 to 4 carbon atoms, a fluorine atom, or a trifluoromethyl group;
n, m, and p each independently represents an integer of 0 to 3; and k and q each independently represents an integer of 0 to 2.

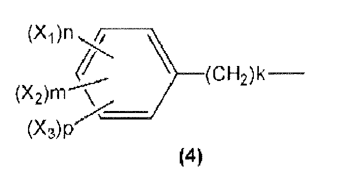

In the formula (4), X₁, X₂, X₃, k, n, m, and p are as defined in the formula (3).

Also, the present invention provides the parakeratosis inhibitor or pore-shrinking agent, comprising, as an active ingredient, one or more compounds selected from the group consisting of glutamic acid derivatives represented by the following formula (1a) and the salts thereof.

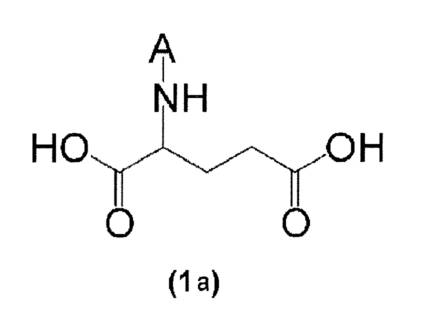

In the formula (1a), A represents a carbamoyl group, a benzyloxycarbonyl group, an alkyl group having 1 to 3 carbon atoms, or an allyl group.

Also, the present invention provides the parakeratosis inhibitor or pore-shrinking agent, comprising, as an active ingredient, one or more compounds selected from the group consisting of N-carbamoylglutamic acid, N-benzyloxycarbonylglutamic acid, N-methylglutamic acid, and the salts thereof.

Also, the present invention provides the parakeratosis inhibitor or pore-shrinking agent, comprising, as an active ingredient, one or more compounds selected from the group consisting of glutamic acid derivatives represented by the formula (2) and the salts thereof.
Also, the present invention provides the parakeratosis inhibitor or pore-shrinking agent, wherein Z is a methyl group or a phenyl group.
Also, the present invention provides the parakeratosis inhibitor or pore-shrinking agent, wherein Y is a hydroxyl group or NR₃R₄.
Also, the present invention provides the parakeratosis inhibitor or pore-shrinking agent, wherein Y is the same as NR₁R₂.

Also, the present invention provides the parakeratosis inhibitor or pore-shrinking agent, wherein at least one of R₁ and R₂, and/or at least one of R₃ and R₄, each independently represents a hydrogen atom, an alkyl group having 1 to 3 carbon atoms, a cycloalkyl group having 3 to 7 carbon atoms, or a benzyl group.
Also, the present invention provides the parakeratosis inhibitor or pore-shrinking agent, wherein at least one of R₁ and R₂, and/or at least one of R₃ and R₄, each independently represents a hydrogen atom, a methyl group, or an ethyl group.
Also, the present invention provides the parakeratosis inhibitor or pore-shrinking agent, wherein R₁ and R₂, and/or R₃ and R₄, independently form the heterocycle having 6 carbon atoms or less in total together with the nitrogen atom to which they are bonded.

Also, the present invention provides the parakeratosis inhibitor or pore-shrinking agent, comprising, as an active ingredient, one or more compounds selected from N-acetyl-N'-methylglutamic acid-1-amide, N-acetyl-N'-ethylglutamic acid-1-amide, N-acetyl-N'-n-propylglutamic acid-1-amide, N-acetyl-N'-benzylglutamic acid-1-amide, N-acetyl-N'-cyclohexylglutamic acid-1-amide, N-acetyl-N'-cyclopentylglutamic acid-1-amide, N-acetylglutamic acid 1-pyrrolidine amide, N-acetylglutamic acid 1-piperidine amide, N-acetylglutamic acid 1-morpholine amide, N-benzoyl-N'-methylglutamic acid-1-amide, N-benzoyl-N'-ethylglutamic acid-1-amide, N-benzoyl-N'-n-propylglutamic acid-1-amide, N-benzoylglutamic acid 1-morpholine amide, N-acetyl-N',N"-dimethylglutamic acid-1,5-diamide, N-acetyl-N',N"-diethylglutamic acid-1,5-diamide, N-acetyl-N',N',N",N"-tetramethylglutamic acid-1,5-diamide, N-acetyl-N',N',N",N"-tetraethylglutamic acid-1,5-diamide, N-acetylglutamic acid bis-1,5-morpholine amide, N-acetylglutamic acid bis-1,5-piperidine amide, and the salts thereof.

Also, the present invention provides the parakeratosis inhibitor or pore-shrinking agent, comprising, as an active ingredient, one or more compounds selected from the group consisting of N-amidinoglutamic acid represented by the following formula (1b) and the salt thereof.

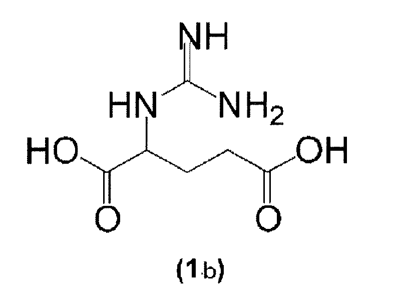

Also, the present invention provides the parakeratosis inhibitor or pore-shrinking agent, comprising, as an active ingredient, one or more compounds selected from the group consisting ofN-amidino-L-glutamic acid (S-2-guanidinoglutaric acid) and the salt thereof.
Also, the present invention provides an external composition for skin, comprising any of the above-mentioned parakeratosis inhibitors or pore-shrinking agents.

Also, the present invention provides a rough skin inhibiting or ameliorating agent, comprising, as an active ingredient, one or more compounds selected from the group consisting of glutamic acid derivatives represented by the following formula (1a) or (2) and the salts thereof.

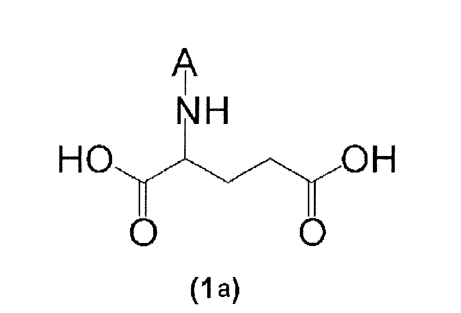

In the formula (1a), A represents a carbamoyl group, a benzyloxycarbonyl group, an alkyl group having 1 to 3 carbon atoms, or an allyl group.

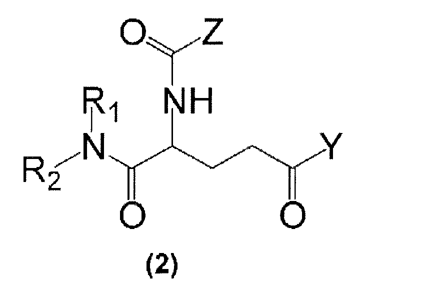

In the formula (2), Z represents an alkyl group having 1 to 4 carbon atoms, an alkenyl group having 2 to 4 carbon atoms, a benzyloxy group, or a group represented by the following formula (3) or (4);
Y represents any of a hydroxyl group, an alkyloxy group having 1 to 3 carbon atoms, an allyloxy group, or NR₃R₄, with the proviso that Y is not NR₃R₄ when Z is a group represented by the formula (4); and
R₁ to R₄ each independently represents a hydrogen atom, an alkyl group having 1 to 3 carbon atoms, an allyl group, a cycloalkyl group having 3 to 7 carbon atoms, a cycloalkenyl group having 3 to 7 carbon atoms, a phenyl group, or a benzyl group, or R₁ and R₂, and/or R₃ and R₄, independently form a heterocycle having 6 carbon atoms or less in total together with the nitrogen atom to which they are bonded, wherein the heterocycle may include an oxygen atom,
in R₁ to R₄, the alkyl group having 1 to 3 carbon atoms, the allyl group, the cycloalkyl group having 3 to 7 carbon atoms, the cycloalkenyl group having 3 to 7 carbon atoms, the phenyl group, the benzyl group, or the heterocycle may have, as a substituent, a hydroxyl group, an alkyloxy group having 1 to 3 carbon atoms, or an allyloxy group, and
in R₁ to R₄, the cycloalkyl group having 3 to 7 carbon atoms, the cycloalkenyl group having 3 to 7 carbon atoms, the phenyl group, the benzyl group, or the heterocycle may have, as a substituent, an alkyl group having 1 to 3 carbon atoms.

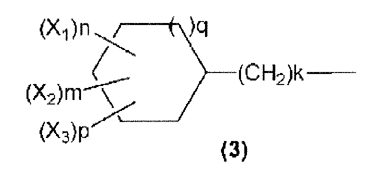

In the formula (3), X₁, X₂, and X₃ each independently represents an alkyl group having 1 to 4 carbon atoms, an alkenyl group having 1 to 4 carbon atoms, an alkyloxy group having 1 to 4 carbon atoms, an alkenyloxy group having 2 to 4 carbon atoms, a hydroxyl group, an amino group, an alkylamino group having 1 to 4 carbon atoms, a fluorine atom, or a trifluoromethyl group;
n, m, and p each independently represents an integer of 0 to 3; and
k and q each independently represents an integer of 0 to 2.

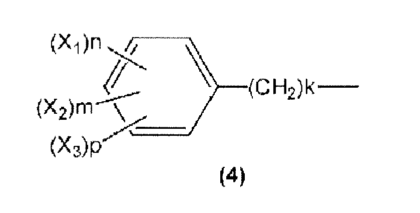

In the formula (4), X₁, X₂, X₃, k, n, m, and p are as defined in the formula (3).

Also, the present invention provides the rough skin inhibiting or ameliorating agent, comprising, as an active ingredient, one or more compounds selected from the group consisting of glutamic acid derivatives represented by the formula (1a) and the salts thereof.
Also, the present invention provides the rough skin inhibiting or ameliorating agent, comprising, as an active ingredient, one or more compounds selected from the group consisting of N-carbamoylglutamic acid, N-benzyloxycarbonylglutamic acid, N-methylglutamic acid, and the salts thereof.

Also, the present invention provides the rough skin inhibiting or ameliorating agent, comprising, as an active ingredient, one or more compounds selected from the group consisting of glutamic acid derivatives represented by the formula (2) and the salts thereof.
Also, the present invention provides the rough skin inhibiting or ameliorating agent, wherein Z is a methyl group or a phenyl group.
Also, the present invention provides the rough skin inhibiting or ameliorating agent, wherein Y is a hydroxyl group or NR₃R₄.
Also, the present invention provides the rough skin inhibiting or ameliorating agent, wherein Y is the same as NR₁R₂.

Also, the present invention provides the rough skin inhibiting or ameliorating agent, wherein at least one of R₁ and R₂, and/or at least one of R₃ and R₄, each independently represents a hydrogen atom, an alkyl group having 1 to 3 carbon atoms, a cycloalkyl group having 3 to 7 carbon atoms, or a benzyl group.
Also, the present invention provides the rough skin inhibiting or ameliorating agent, wherein at least one of R₁ and R₂, and/or at least one of R₃ and R₄, each independently represents a hydrogen atom, a methyl group, or an ethyl group.
Also, the present invention provides the rough skin inhibiting or ameliorating agent, wherein R₁ and R₂, and/or R₃ and R₄, independently form the heterocycle having 6 carbon atoms or less in total together with the nitrogen atom to which they are bonded.

Also, the present invention provides the rough skin inhibiting or ameliorating agent, being one or more compounds selected from N-acetyl-N'-methylglutamic acid-1-amide, N-acetyl-N'-ethylglutamic acid-1-amide, N-acetyl-N'-n-propylglutamic acid-1-amide, N-acetyl-N'-benzylglutamic acid-1-amide, N-acetyl-N'-cyclohexylglutamic acid-1-amide, N-acetyl-N'-cyclopentylglutamic acid-1-amide, N-acetylglutamic acid 1-pyrrolidine amide, N-acetylglutamic acid 1-piperidine amide, N-acetylglutamic acid 1-morpholine amide, N-benzoyl-N'-methylglutamic acid-1-amide, N-benzoyl-N'-ethylglutamic acid-1-amide, N-benzoyl-N'-n-propylglutamic acid-1-amide, N-benzoylglutamic acid 1-morpholine amide, N-acetyl-N',N"-dimethylglutamic acid-1,5-diamide, N-acetyl-N',N"-diethylglutamic acid-1,5-diamide, N-acetyl-N',N',N",N"-tetramethylglutamic acid-1,5-diamide, N-acetyl-N',N',N",N"-tetraethylglutamic acid-1,5-diamide, N-acetylglutamic acid bis-1,5-morpholine amide, N-acetylglutamic acid bis-1,5-piperidine amide, and the salts thereof.

Also, the present invention provides an external composition for skin, comprising the rough skin inhibiting or ameliorating agent.
Also, the present invention provides an external composition for skin comprising one or more compounds selected from the group consisting of glutamic acid derivatives represented by the formula (1a) or (2) and the salts thereof.

### EFFECT OF THE INVENTION

The present invention provides a novel parakeratosis inhibitor, a novel pore-shrinking agent, and a novel rough skin inhibiting or ameliorating agent, which have excellent effects, cause no sensory irritation, and are high in safety with use of a glutamic acid derivative having a specific structure or the salt thereof. In addition, by incorporating such glutamic acid derivative or the salt thereof, an external composition for skin which has functions such as inhibiting parakeratosis, shrinking pores, and inhibiting/ameliorating rough skin can be obtained.

### BRIEF DESCRIPTION OF THE DRAWINGS

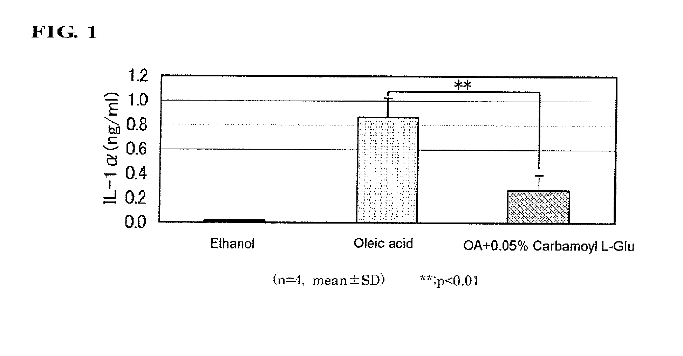

Fig. 1 shows the result of the amount of cytokine (IL-1α) measured in the case where ethanol, oleic acid, or both of oleic acid and N-carbamoyl-L-glutamic acid was added individually to human normal keratinocyte culture system.

### BEST MODE FOR CARRYING OUT THE INVENTION

The parakeratosis inhibitor and pore-shrinking agent according to the present invention comprises, as an active ingredient, one or more compounds selected from the group consisting of glutamic acid derivatives represented by the following formula (1) or (2) and the salts thereof.

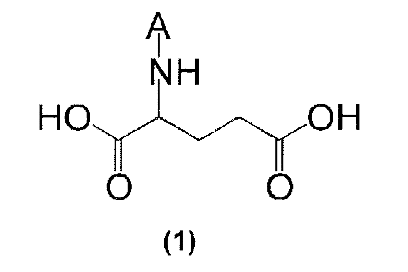

In the formula (1), A represents a carbamoyl group, a benzyloxycarbonyl group, an alkyl group having 1 to 3 carbon atoms, an allyl group, or an amidino group.

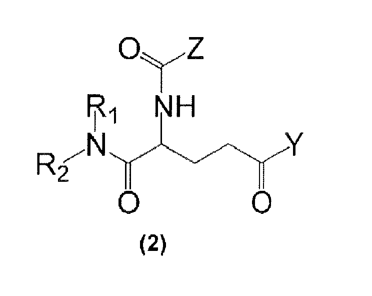

In the formula (2), Z represents an alkyl group having 1 to 4 carbon atoms, an alkenyl group having 2 to 4 carbon atoms, a benzyloxy group, or a group represented by the following formula (3) or (4);
Y represents a hydroxyl group, an alkyloxy group having 1 to 3 carbon atoms, an allyloxy group, or NR₃R₄; with the proviso that Y is not NR₃R₄ when Z is a group represented by the formula (4); and
R₁ to R₄ each independently represents a hydrogen atom, an alkyl group having 1 to 3 carbon atoms, an allyl group, a cycloalkyl group having 3 to 7 carbon atoms, a cycloalkenyl group having 3 to 7 carbon atoms, a phenyl group, or a benzyl group, or R₁ and R₂ and R₃ and R₄ independently form a heterocycle having 6 carbon atoms or less in total together with the nitrogen atom to which they are bonded, wherein
the heterocycle may include an oxygen atom,
in R₁ to R₄, the alkyl group having 1 to 3 carbon atoms, the allyl group, the cycloalkyl group having 3 to 7 carbon atoms, the cycloalkenyl group having 3 to 7 carbon atoms, the phenyl group, the benzyl group, or the heterocycle may have, as a substituent, a hydroxyl group, an alkyloxy group having 1 to 3 carbon atoms, or an allyloxy group, and
in R₁ to R₄, the cycloalkyl group having 3 to 7 carbon atoms, the cycloalkenyl group having 3 to 7 carbon atoms, the phenyl group, the benzyl group, or the heterocycle may have, as a substituent, an alkyl group having 1 to 3 carbon atoms.

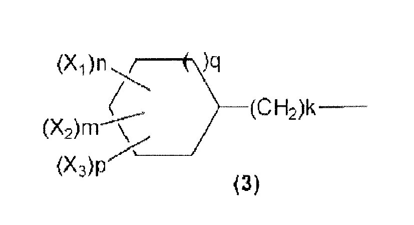

In the formula (3), X₁, X₂, and X₃ each independently represents an alkyl group having 1 to 4 carbon atoms, an alkenyl group having 1 to 4 carbon atoms, an alkyloxy group having 1 to 4 carbon atoms, an alkenyloxy group having 2 to 4 carbon atoms, a hydroxyl group, an amino group, an alkylamino group having 1 to 4 carbon atoms, a fluorine atom, or a trifluoromethyl group;
n, m, and p each independently represents an integer of 0 to 3; and
k and q each independently represents an integer of 0 to 2.

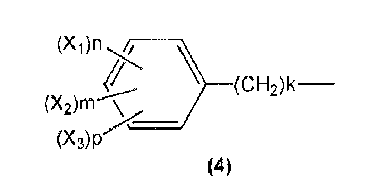

In the formula (4), X₁, X₂, X₃, k, n, m, and p are as defined in the formula (3).

Also, the rough skin inhibiting/ameliorating agent according to the present invention comprises, as an active ingredient, one or more compounds selected from the group consisting of glutamic acid derivatives represented by the following formula (1a) or (2) and the salts thereof.

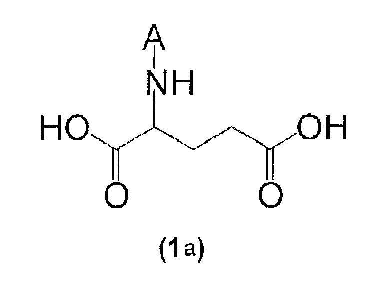

In the formula (1a), A represents a carbamoyl group, a benzyloxycarbonyl group, an alkyl group having 1 to 3 carbon atoms, or an allyl group.

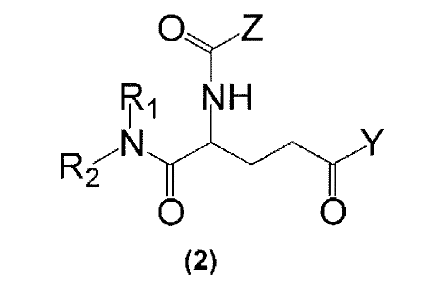

In the formula (2), Z, Y, and R₁ to R₄ are as defined above.

The glutamic acid derivatives represented by the formula (1) will be described first.
Preferable examples of the glutamic acid derivatives represented by the formula (1) include glutamic acid derivatives represented by the following formula (1a).

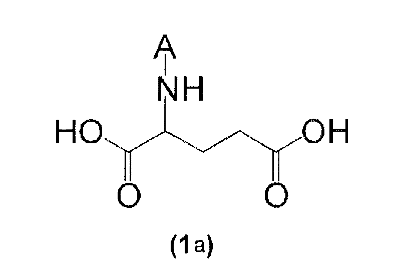

In the formula (1a), A represents a carbamoyl group, a benzyloxycarbonyl group, an alkyl group having 1 to 3 carbon atoms, or an allyl group.

Specifically, examples of the alkyl group having 1 to 3 carbon atoms include methyl, ethyl, n-propyl, and iso-propyl groups.
In the glutamic acid derivatives represented by the formula (1a), the glutamic acid as a main structure may be any of D-, L-, and DL-forms, and the mixing ratio in DL-form does not be limited. Though the effects of the present invention is good in any forms, in terms of the ease of obtaining the glutamic acid derivatives of the present invention, the glutamic acid is preferably L-form.

Examples of the glutamic acid derivatives of the formula (1a) include N-carbamoyl-L-glutamic acid, N-benzyloxycarbonyl-L-glutamic acid, N-methyl-L-glutamic acid, N-ethyl-L-glutamic acid, N-n-propyl-L-glutamic acid, N-iso-propyl-L-glutamic acid, N-allyl-L-glutamic acid, N-carbamoyl-DL-glutamic acid, N-benzyloxycarbonyl-DL-glutamic acid, N-methyl-DL-glutamic acid, N-ethyl-DL-glutamic acid, N-n-propyl-DL-glutamic acid, N-iso-propyl-DL-glutamic acid, N-allyl-DL-glutamic acid, N-carbamoyl-D-glutamic acid, N-benzyloxycarbonyl-D-glutamic acid, N-methyl-D-glutamic acid, N-ethyl-D-glutamic acid, N-n-propyl-D-glutamic acid, N-iso-propyl-D-glutamic acid, and N-allyl-D-glutamic acid.

Among the glutamic acid derivatives represented by the formula (1a), the most preferable examples include N-carbamoyl-L-glutamic acid, N-benzyloxycarbonyl-L-glutamic acid, N-methyl-L-glutamic acid, and the salts thereof. These derivatives are the most excellent, among the glutamic acid derivatives represented by the formula (1a), in the effects of inhibiting parakeratosis, shrinking pores, and inhibiting/ameliorating rough skin. In addition, these derivatives are favorably soluble in preparations, have high safety, and provide the external compositions for skin, to which they blended, excellent in these effects. Thus, these derivatives are the most excellent in terms of achieving the objects of the present invention.

Another preferable example of the glutamic acid derivatives represented by the formula (1) includes N-amidinoglutamic acid represented by the following formula (1b).

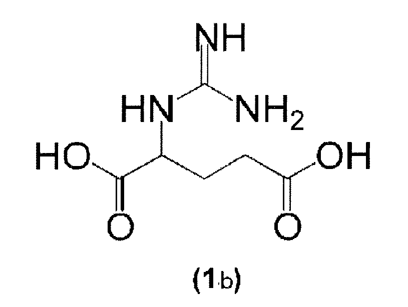

The N-amidinoglutamic acid of the formula (1b) is a compound having a structure wherein an amino group of the glutamic acid has an amidino group. The compound is known as N-amidinoglutamic acid, 2-guanidinoglutaric acid, or 2-guanidino pentane-1,5-dicarboxylic acid.
In the N-amidinoglutamic acid of the present invention, the glutamic acid as a main structure may be any of D-, L-, and DL-forms, and the mixing ratio in DL-form does not be limited.
Among these, N-amidino-L-glutamic acid (S-2-guanidinoglutaric acid), in which the glutamic acid is L-form, is particularly preferable because of the good effects.

Next, the glutamic acid derivatives represented by the formula (2) will be described.
In the formula (2), Z represents an alkyl group having 1 to 4 carbon atoms, an alkenyl group having 2 to 4 carbon atoms, a benzyloxy group, or a group represented by the formula (3) or (4). The glutamic acid derivatives of the present invention, represented by the formula (2), are acyl glutamic acid derivatives having Z moiety.

In Z, an alkyl group having 1 to 4 carbon atoms can be liner, branched, or cyclic.
An alkenyl group having 2 to 4 carbon atoms can be liner, branched, or cyclic, and has one or more double bonds in an arbitrary position.

When Z is an alkyl group having 1 to 4 carbon atoms or an alkenyl group having 2 to 4 carbon atoms, examples of the acyl groups represented by -CO-Z include acetyl, propanoyl, propenoyl, butanoyl, 2-butenoyl, 3-butenoyl, 2-methylpropanoyl, 2-methyl-3-propenoyl, pentanoyl, 2-methylbutanoyl, 2-methyl-2-butenoyl, 2-methyl-3-butenoyl, 2-ethylpropenoyl, 3-methylbutanoyl, 3-methyl-2-butenoyl, 3-methyl-3-butenoyl, 2,2-dimethylpropanoyl, 2-pentenoyl, 3-pentenoyl, 4-pentenoyl, 2,4-pentadienoyl, cyclopropanecarbonyl, cyclobutanecarbonyl, cyclopropylacetyl, 1'-cyclopropenecarbonyl, 2'-cyclopropenecarbonyl, 1'-cyclobutenecarbonyl, 2'-cyclobutenecarbonyl, 1',3'-cyclobutadiene carbonyl, 1'-cyclopropeneacetyl, and 2'-cyclopropeneacetyl groups.
Among these, an acetyl group is preferable because the effects of the present invention and the solubility are good.

When Z is a benzyloxy group, the glutamic acid derivative represented by the formula (2) is a benzyloxycarbonyl glutamic acid derivative.

In the formula (3), X₁, X₂, and X₃ each independently represents an alkyl group having 1 to 4 carbon atoms, an alkenyl group having 1 to 4 carbon atoms, an alkyloxy group having 1 to 4 carbon atoms, an alkenyloxy group having 2 to 4 carbon atoms, a hydroxyl group, an amino group, an alkylamino group having 1 to 4 carbon atoms, a fluorine atom, or a trifluoromethyl group; n, m, and p each independently represents an integer of 0 to 3; and k and q each independently represents an integer of 0 to 2. In this context, in the formula (3), the total sum of n+m+p is up to 6, depending on q.

In X₁, X₂, and X₃, the alkyl group having 1 to 4 carbon atoms is a liner or branched alkyl group, and examples thereof include methyl, ethyl, n-propyl, iso-propyl, n-butyl, iso-butyl, tert-butyl, and 1-methylpropyl groups.
The alkenyl of alkenyl group having 1 to 4 carbon atoms can be liner or branched and has one or more double bonds. It may have a double bond between itself and the cycloalkyl moiety of the formula (3).

The alkyloxy group having 1 to 4 carbon atoms is a liner or branched alkyloxy group, and examples thereof include methoxy, ethoxy, n-propyloxy, iso- propyloxy, n-butoxy, iso-butoxy, tert-butoxy, and 2-methylpropyloxy groups.
The alkenyl of alkenyloxy group having 2 to 4 carbon atoms can be liner or branched and has one or more double bonds.

In X₁, X₂, and X₃, the alkylamino group having 1 to 4 carbon atoms is a mono- or di-alkylamino group having one or two liner or branched alkyl groups. Examples thereof include N-methylamino, N-ethylamino, N-n-propylamino, N-iso-propylamino, N-n-butylamino, N-iso-butylamino, N-tert-butylamino, N-(1-methylpropyl)amino, N,N-dimethylamino, N-ethyl-N-methylamino, N-methyl-N-n-propylamino, N-methyl-N-iso-propylamino, N-n-butyl-N-methylamino, N-iso-butyl-N-methylamino, N-tert-butyl-N-methylamino, N-(1-methylpropyl)-N-methylamino, N-ethyl-N-methylamino, N,N-diethylamino, N-ethyl-N-n-propylamino, N-ethyl-N-iso-propylamino, N-n-butyl-N-ethylamino, N-iso-butyl-N-ethylamino, N-tert-butyl-N-ethylamino, N-ethyl-N-(1-methylpropyl)amino, N-methyl-N-n-propylamino, N-ethyl-N-n-propylamino, N,N-di(n-propyl)amino, N-n-propyl-N-iso-propylamino, N-n-butyl-N-n-propylamino, N-iso-butyl-N-n-propylamino, N-tert-butyl-N-n-propylamino, N-(1-methylpropyl)-N-n-propylamino,

N-methyl-N-iso-propylamino, N-ethyl-N-iso-propylamino, N-iso-propyl-N-n-propylamino, N,N-di(iso-propyl)amino, N-n-butyl-N-iso-propylamino, N-iso-butyl-N-iso-propylamino, N-tert-butyl-N-iso-propylamino, N-(1-methylpropyl)-N-iso-propylamino, N-n-butyl-N-methylamino, N-n-butyl-N-ethylamino, N-n-butyl-N-n-propylamino, N-n-butyl-N-iso-propylamino, N,N-di(n-butyl)amino, N-n-butyl-N-iso-butylamino, N-n-butyl-N-tert-butylamino, N-n-butyl-N-(1-methylpropyl)amino, N-iso-butyl-N-methylamino, N-iso-butyl-N-ethylamino, N-iso-butyl-N-n-propylamino, N-n-butyl-N-iso-propylamino, N,N-di(iso-butyl)amino, N-iso-butyl-N-ter-butylamino,

N-iso-butyl-N-tert-butylamino, N-iso-butyl-N-1-methylpropylamino, N-ter-butyl-N-methylamino, N-ter-butyl-N-ethylamino, N-ter-butyl-N-n-propylamino, N-ter-butyl-N-iso-propylamino, N,N-di(ter-butyl)amino, N-ter-butyl-N-iso-butylamino, N-n-butyl-N-tert-butylamino, N-ter-butyl-N-(1-methylpropyl)amino, N-methyl-N-(1-methylpropyl)amino, N-ethyl-N-(1-methylpropyl)amino, N-(1-methylpropyl)-N-n-propylamino, N-(1-methylpropyl)-N-iso-propylamino, N-n-butyl-1-methylpropylamino, N-iso-butyl-N-(1-methylpropyl)amino, N-tert-butyl-N-(1-methylpropyl)amino, and N,N-di(1-methylpropyl)amino groups.

As X₁ to X₃ of the formula (3), an alkyl group having 1 to 4 carbon atoms, an alkenyl group having 1 to 4 carbon atoms, an alkoxy group having 1 to 4 carbon atoms, or a hydroxyl group is preferable because the effects of the present invention are good. In addition, a methyl, ethyl, methoxy, ethoxy, or hydroxyl group is more preferable because the solubility is good. The case where n, m, and p are zero is also preferable in terms of the effects of the present invention and the solubility.

The glutamic acid derivative of the present invention is: a cycloalkylcarbonylglutamic acid derivative corresponding to q when k of the formula (3) is zero; a cycloalkylmethylcarbonylglutamic acid derivative corresponding to q when k of the formula (3) is 1, or a cycloalkylethylcarbonylglutamic acid derivative corresponding to q when k of the formula (3) is 2. The formula (3) has: a cyclopentyl moiety when q is zero; a cyclohexyl moiety when q is 1; and a cycloheptyl moiety when q is 2.
Among these, it is preferred that k is zero or q is 1 because the effects of the present invention are good.

When Z is the group represented by the formula (3), preferable examples of the -CO-Z group include cyclohexanecarbonyl, 4-methoxycyclohexanecarbonyl, 3-methoxycyclohexanecarbonyl, 2-methoxycyclohexanecarbonyl, 4-hydroxycyclohexanecarbonyl, 3-hydroxycyclohexanecarbonyl, 2-hydroxycyclohexanecarbonyl, cyclohexylacetyl, 4-methoxycyclohexylacetyl, 3-methoxycyclohexylacetyl, 2-methoxycyclohexylacetyl, 4-hydroxycyclohexylacetyl, 3-hydroxycyclohexylacetyl, 2-hydroxycyclohexylacetyl, cyclohexylpropionyl, 4-methoxycyclohexylpropionyl, 3-methoxycyclohexylpropionyl, 2-methoxycyclohexylpropionyl, 4-hydroxycyclohexylpropionyl, 3-hydroxycyclohexylpropionyl, and 2-hydroxycyclohexylpropionyl groups, and a cyclohexanecarbonyl group is particularly preferable.

In the formula (4), X₁, X₂, X₃, k, n, m, and p are as defined in the formula (3), and the preferable examples thereof are also as defined in the formula (3). However, in the formula (4), the total sum of n+m+p is up to 5.
In the case that Z is the group represented by the formula (4), the -CO-Z group is: a benzoyl group corresponding to X₁ to X₃ when k is zero; a phenylacetyl group corresponding to X₁ to X₃ when k is 1; or a phenylpropionyl group corresponding to X₁ to X₃ when k is 2. In the formula (4), it is preferred that k is zero because the effects of the present invention are good.

When Z is the formula (4), preferable examples of the -CO-Z group include benzoyl, p-anisoyl (4-methoxybenzoyl), m-anisoyl (3-methoxybenzoyl), o-anisoyl (2-methoxybenzoyl), 4-ethoxybenzoyl, 3-ethoxybenzoyl, 2-ethoxybenzoyl, 4-propoxybenzoyl, 3-propoxybenzoyl, 2-propoxybenzoyl, 2,4-dimethoxybenzoyl, 3,4-dimethoxybenzoyl, 3,4,5-trimethoxybenzoyl, 2,3,4-trimethoxybenzoyl, 2-hydroxybenzoyl (salicyl), 3-hydroxybenzoyl, 4-hydroxybenzoyl, 2,4-dihydroxybenzoyl, 3,4-dihydroxybenzoyl, 3,4,5-trihydroxybenzoyl (galloyl), 2-hydroxy-4-methoxybenzoyl, 3-hydroxy-4-methoxybenzoyl, 4-hydroxy-3-methoxybenzoyl, 2-methylbenzoyl (o-toluyl), 3-methylbenzoyl (m-toluyl), 4-methylbenzoyl (p-toluyl), 2-ethylbenzoyl, 3-ethylbenzoyl, 4-ethylbenzoyl, 2-propylbenzoyl, 3-propylbenzoyl, 4-propylbenzoyl,

phenylacetyl, 4-methoxyphenylacetyl, 3-methoxyphenylacetyl, 2-methoxyphenylacetyl, 4-ethoxyphenylacetyl, 3-ethoxyphenylacetyl, 2-ethoxyphenylacetyl, 4-propoxyphenylacetyl, 3-propoxyphenylacetyl, 2-propoxyphenylacetyl, 2,4-dimethoxyphenylacetyl, 3,4-dimethoxyphenylacetyl, 3,4,5-trimethoxyphenylacetyl, 2-hydroxyphenylacetyl, 3-hydroxyphenylacetyl, 4-hydroxyphenylacetyl, N-2,4-dihydroxyphenylacetyl, N-3,4-dihydroxyphenylacetyl, N-3,4,5-trihydroxyphenylacetyl, 2-hydroxy-4-methoxyphenylacetyl, 3-hydroxy-4-methoxyphenylacetyl, 4-hydroxy-3-methoxyphenylacetyl, 2-methylphenylacetyl, 3-methylphenylacetyl, 4-methylphenylacetyl, 2-ethylphenylacetyl, 3-ethylphenylacetyl, 4-ethylphenylacetyl, 2-propylphenylacetyl, 3-propylphenylacetyl, 4-propylphenylacetyl,

phenylpropionyl, 4-methoxyphenylpropionyl, 3-methoxyphenylpropionyl, 2-methoxyphenylpropionyl, 4-ethoxyphenylpropionyl, 3-ethoxyphenylpropionyl, 2-ethoxyphenylpropionyl, 4-propoxyphenylpropionyl, 3-propoxyphenylpropionyl, 2-propoxyphenylpropionyl, 2,4-dimethoxyphenylpropionyl, 3,4-dimethoxyphenylpropionyl, 3,4,5-trimethoxyphenylpropionyl, 2-hydroxyphenylpropionyl, 3-hydroxyphenylpropionyl, 4-hydroxyphenylpropionyl, N-2,4-dihydroxyphenylpropionyl, N-3,4-dihydroxyphenylpropionyl, N-3,4,5-trihydroxyphenylpropionyl, 2-hydroxy-4-methoxyphenylpropionyl, 3-hydroxy-4-methoxyphenylpropionyl, 4-hydroxy-3-methoxyphenylpropionyl, 2-methylphenylpropionyl, 3-methylphenylpropionyl, 4-methylphenylpropionyl, 2-ethylphenylpropionyl, 3-ethylphenylpropionyl, 4-ethylphenylpropionyl, 2-propylphenylpropionyl, 3-propylphenylpropionyl, and 4-propylphenylpropionyl groups. Among these, a benzoyl group is preferable because the effects of the present invention are the best.

In the formula (2), Y represents a hydroxyl group, an alkyloxy group having 1 to 3 carbon atoms, an allyloxy group, or NR₃R₄.
When Y is a hydroxyl group, the glutamic acid derivative of the formula (2) is an 1-amidated compound corresponding to R₁ and R₂. Thus, the glutamic acid derivative of the formula (2) is an acylglutamic acid-1-amide corresponding to Z, R₁, and R₂.

When the Y is an alkyloxy group having 1 to 3 carbon atoms or an allyloxy group, the glutamic acid derivative of the formula (2) is a 5-esterified compound. Thus, the glutamic acid derivative of the formula (2) is an acylglutamic acid-1-monoamide-5-ester corresponding to Y, Z, R₁, and R₂. In this context, specific examples ofY include methoxy, ethoxy, n-propoxy, iso-propoxy, and allyloxy groups.

When Y is NR₃R₄, the glutamic acid derivative of the formula (2) is an 1,5-diamidated compound corresponding to R₁ to R₄. Thus, the glutamic acid derivative of the formula (2) is an acylglutamic acid-1,5-diamide corresponding to Y, Z, and R₁ to R₄. However, when Z is the formula (4), Y is not NR₃R₄.

For Y in the formula (2), a hydroxyl group or NR₃R₄ is preferable because of the good stability.
In the formula (2), R₁ to R₄ can each independently represent a hydrogen atom, an alkyl group having 1 to 3 carbon atoms, an allyl group, a cycloalkyl group having 3 to 7 carbon atoms, a cycloalkenyl group having 3 to 7 carbon atoms, a phenyl group, or a benzyl group. Alternatively, R₁ and R₂, and/or R₃ and R₄, may independently form a heterocycle having 6 carbon atoms or less in total together with the nitrogen atom to which they are bonded. The heterocycle may include an oxygen atom.

In R₁ to R₄, the alkyl group having 1 to 3 carbon atoms, the allyl group, the cycloalkyl group having 3 to 7 carbon atoms, the cycloalkenyl group having 3 to 7 carbon atoms, the phenyl group, the benzyl group, and the heterocycle may have a hydroxyl group, an alkyloxy group having 1 to 3 carbon atoms, or an allyloxy group as a substituent.
Also, in R₁ to R₄, the cycloalkyl group having 3 to 7 carbon atoms, the cycloalkenyl group having 3 to 7 carbon atoms, the phenyl group, the benzyl group, and the heterocycle may have an alkyl group having 1 to 3 carbon atoms as a substituent.

When both R₁ and R₂ are hydrogen atoms and Y is a hydroxyl group, the glutamic acid derivative of the present invention is acylglutamic acid-1-amide or acylglutamine amide corresponding to Z.

In R₁ to R₄, examples of the alkyl group having 1 to 3 carbon atoms include methyl, ethyl, n-propyl, and iso-propyl groups.
Also, in R₁ to R₄, the alkyl group having 1 to 3 carbon atoms and the allyl group may have a hydroxyl group, an alkyloxy group having 1 to 3 carbon atoms, or an allyloxy group as a substituent.
Examples thereof include 2-hydroxyethyl, 2-hydroxypropyl, 3-hydroxypropyl, 1-hydroxy-1-methylethyl, 2-hydroxy-1-methylethyl, 2,3-dihydroxypropyl, 2,2,3-trihydroxypropyl, 2,1'-dihydroxy-1-methylethyl, 2-methoxyethyl, 2-methoxypropyl, 3-methoxypropyl, 1-methoxy-1-methylethyl, 2-methoxy-1-methylethyl, 2,3-dimethoxypropyl, 2,2,3-trimethoxypropyl, 2,1'-dimethoxy-1-methylethyl, 2-ethoxyethyl, 2-ethoxypropyl, 3-ethoxypropyl, 1-ethoxy-1-methylethyl, 2-ethoxy-1-methylethyl, 2,3-diethoxypropyl, 2,2,3-triethoxypropyl, 2,1'-diethoxy-1-methylethyl, 2-n-propoxyethyl, 2-n-propoxypropyl, 3-n-propoxypropyl, 1-n-propoxy-1-methylethyl, 2-n-propoxy-1-methylethyl, 2,3-di-n-propoxypropyl, 2,2,3-tri-n-propoxypropyl, 2,1'-di-n-propoxy-1-methylethyl, 2-iso-propoxyethyl, 2-iso-propoxy-iso-propyl, 3-iso-propoxypropyl, 1-iso-propoxy-1-methylethyl, 2-iso-propoxy-1-methylethyl, 2,3-di-iso-propoxypropyl, 2,2,3-tri-iso-propoxypropyl, 2,1'-di-iso-propoxy-1-methylethyl, 2-allyloxyethyl, 2-allyloxy-n-propyl, 3-allyloxypropyl, 1-allyloxy-1-methylethyl, 2-allyloxy-1-methylethyl, 2,3-diallyloxypropyl, 2,2,3-triallyloxypropyl, 2,1'-diallyloxy-1-methylethyl, 2-hydroxy-3-methoxypropyl, and 3-hydroxy-2-methoxypropyl groups.

In R₁ to R₄, the cycloalkyl group having 3 to 7 carbon atoms means a saturated hydrocarbon group having 3 to 7 carbon atoms in total, which forms a saturated hydrocarbon ring at least in part.
Also, in R₁ to R₄, the cycloalkenyl group having 3 to 7 carbon atoms means a unsaturated hydrocarbon group having 3 to 7 carbon atoms in total , which forms a unsaturated carbon ring having one or more double bonds at least in part.

Examples of the cycloalkyl group having 3 to 7 carbon atoms or the cycloalkenyl group having 3 to 7 carbon atoms include cyclopropyl, cyclopropylmethyl, 1-methylcyclopropyl, 2-methylcyclopropyl, 3-methylcyclopropyl, 1-cyclopropylethyl, 2-cyclopropylethyl, 1,2-dimethylcyclopropyl, 2,2-dimethylcyclopropyl, 2,3-dimethylcyclopropyl, 1-cyclopropylpropyl, 2-cyclopropylpropyl, 3-cyclopropylpropyl, (2'-methylcyclopropyl)methyl, (2'-ethylcyclopropyl)methyl, 1-ethylcyclopropyl, 2-ethylcyclopropyl, 1,2,3-trimethylcyclopropyl, 2,2,3-trimethylcyclopropyl, 1,2,2-trimethylcyclopropyl, (1',2'-dimethylcyclopropyl)methyl, (2',2'-dimethylcyclopropyl)methyl, (2',3'-dimethylcyclopropyl)methyl, 1-(1'-methylcyclopropyl)ethyl, 1-(2'-methylcyclopropyl)ethyl, 2-cyclopropylpropyl, 2-(2'-methylcyclopropyl)ethyl, 1-(1'-methylcyclopropyl)propyl, 1-(2'-methylcyclopropyl)propyl, 2-ethyl-1-methylcyclopropyl, 2-ethyl-2-methylcyclopropyl, 3-cyclopropylbutyl, 2-cyclopropylbutyl, 1-cyclopropylbutyl, 2-cyclopropyl-1-methylethyl, 3-(2'-methylcyclopropyl)propyl, 1-(2'-propylcyclopropyl)methyl, 3-cyclopropyl-2-methylpropyl, 3-cyclopropyl-1-methylpropyl, 3-cyclopropylpentyl, 3-(2'-ethylcyclopropyl)ethyl, 1,2-diethylcyclopropyl, 2,2-diethylcyclopropyl,

2-cyclopropyl-1-ethylethyl, 2-(2',2'-dimethylcyclopropyl)ethyl, 2-(2',3'-dimethylcyclopropyl)ethyl, 2-(2',2'-dimethylcyclopropyl)butyl, 2-(2',3'-dimethylcyclopropyl)butyl, 2-(2'-methylcyclopropyl)-1-methylethyl, 2-cyclopropyl-1-methylbutyl, tetramethylcyclopropyl, (2',2',3'-trimethylcyclopropyl)methyl, 1-(2',2'-dimethylcyclopropyl)ethyl, 1-(2',3'-dimethylcyclopropyl)ethyl, 1-(2',2'-dimethylcyclopropyl)-1-methylmethyl, 1-(2',3'-dimethylcyclopropyl)-1-methylmethyl, 1-(3'-methylcyclopropyl)-1-methylethyl, 1-(2'-methylcyclopropyl)-1-methylethyl, 1-(2'-ethylcyclopropyl)-1-methylmethyl, 1-cyclopropyl-1-methylethyl, cyclobutyl, cyclobutylmethyl, 1-methylcyclobutyl, 2-methylcyclobutyl, 3-methylcyclobutyl, 1,2-dimethylcyclobutyl, 1,3-dimethylcyclobutyl, 2,2-dimethylcyclobutyl, 2,3-dimethylcyclobutyl, 2,4-dimethylcyclobutyl, 3,3-dimethylcyclobutyl, 1-ethylcyclobutyl, 2-ethylcyclobutyl, 3-ethylcyclobutyl, 1-cyclobutylethyl, 2-cyclobutylethyl, 1-cyclobutyl-1-methylmethyl, (2',2'-dimethylcyclobutyl)methyl, (2',3'-dimethylcyclobutyl)methyl, (3',3'-dimethylcyclobutyl)methyl, (2',4'-dimethylcyclobutyl)methyl, 1-(2'-methylcyclobutyl)ethyl, 1-(3'-methylcyclobutyl)ethyl,

1,2,2-trimethylcyclobutyl, 1,2,3-trimethylcyclobutyl, 1,3,3-trimethylcyclobutyl, 1,2,4-trimethylcyclobutyl, 2,2,3-trimethylcyclobutyl, 2,3,3-trimethylcyclobutyl, 2,3,4-trimethylcyclobutyl, (2'-ethylcyclobutyl)methyl, (3'-ethylcyclobutyl)methyl, 1-cyclobutylpropyl, 2-cyclobutylpropyl, 3-cyclobutylpropyl, 1-propylcyclobutyl, 2-propylcyclobutyl, 1-ethyl-2-methylcyclobutyl, 2-ethyl-2-methylcyclobutyl, 1-ethyl-3-methylcyclobutyl, 3-ethyl-3-methylcyclobutyl, 2-ethyl-3-methylcyclobutyl, 3-ethyl-2-methylcyclobutyl, 2-ethyl-1-methylcyclobutyl, 3-ethyl-1-methylcyclobutyl, 2-ethyl-4-methylcyclobutyl, 4-ethyl-2-methylcyclobutyl, 2-cyclobutyl-1-methylethyl, 2-(2'-methylcyclobutyl)ethyl, 2-(3'-methylcyclobutyl)ethyl, cyclopentyl, 1-methyl-2-propylcyclobutyl, 1-methyl-3-propylcyclobutyl, 2-methyl-2-propylcyclobutyl, 3-methyl-3-propylcyclobutyl, 2-methyl-3-propylcyclobutyl, 2-methyl-1-propylcyclobutyl, 3-methyl-1-propylcyclobutyl, 2-methyl-4-propylcyclobutyl, cyclopentyl, cyclopentylmethyl, (2'-methylcyclopentyl)methyl, (3'-methylcyclopentyl)methyl, 1-cyclopentylethyl, 2-cyclopentylethyl, 1,2-dimethylcyclopentyl, 2,2-dimethylcyclopentyl, 2,3-dimethylcyclopentyl,

3,3-dimethylcyclopentyl, 1,3-dimethylcyclopentyl, 2,5-dimethylcyclopentyl, 2,4-dimethylcyclopentyl, 3,5-dimethylcyclopentyl, 3,4-dimethylcyclopentyl, 1-cyclopentyl-1-methylmethyl, cyclohexyl, cyclohexylmethyl, 1-methylcyclohexyl, 2-methylcyclohexyl, 3-methylcyclohexyl, cycloheptyl, cyclopropylenyl, cyclopropylenylmethyl, cyclobutenyl, cyclobutadienyl, 1-cyclopentenyl, 2-cyclopentenyl, 3-cyclopentenyl, cyclopentadienyl, 2-methylcyclopentadienyl, 3-methylcyclopentadienyl, 2,2-dimethyl-1-cyclopentenyl, 2,3-dimethyl-1-cyclopentenyl, 2,4-dimethyl-1-cyclopentenyl, 2,5-dimethyl-1-cyclopentenyl, 3,3-dimethyl-1-cyclopentenyl, 3,4-dimethyl-1-cyclopentenyl, 3,5-dimethyl-1-cyclopentenyl, 4,4-dimethyl-1-cyclopentenyl, 4,5-dimethyl-1-cyclopentenyl, 5,5-dimethyl-1-cyclopentenyl, 1,2-dimethyl-2-cyclopentenyl, 1,3-dimethyl-2-cyclopentenyl, 1,4-dimethyl-2-cyclopentenyl, 1,5-dimethyl-2-cyclopentenyl, 2,3-dimethyl-2-cyclopentenyl, 2,4-dimethyl-2-cyclopentenyl, 2,5-dimethyl-2-cyclopentenyl, dimethylcyclopentadienyl, 3,4-dimethyl-2-cyclopentenyl, 3,5-dimethyl-2-cyclopentenyl, 4,4-dimethyl-2-cyclopentenyl, 4,5-dimethyl-2-cyclopentenyl, 5,5-dimethyl-2-cyclopentenyl,

cyclopentenylmethyl, 1,2-dimethyl-3-cyclopentenyl, 1,3-dimethyl-3-cyclopentenyl, 2,2-dimethyl-3-cyclopentenyl, 2,3-dimethyl-3-cyclopentenyl, 2,4-dimethyl-3-cyclopentenyl, 2,5-dimethyl-3-cyclopentenyl, 3,4-dimethyl-3-cyclopentenyl, 3,5-dimethyl-3-cyclopentenyl, 1-(1'-cyclopentenyl)ethyl, 1-(2'-cyclopentenyl)ethyl, 1-(3'-cyclopentenyl)ethyl, 2-(1'-cyclopentenyl)ethyl, 2-(2'-cyclopentenyl)ethyl, 2-(3'-cyclopentenyl)ethyl, 1-cyclohexenyl, 2-cyclohexenyl, 3-cyclohexenyl, 1,3-cyclohexadienyl, 1,4-cyclohexadienyl, 1,5-cyclohexadienyl, 2,4-cyclohexadienyl, 2,5-cyclohexadienyl, 2-methyl-1-cyclohexenyl, 3-methyl-1-cyclohexenyl, 4-methyl-1-cyclohexenyl,

5-methyl-1-cyclohexenyl, 6-methyl-1-cyclohexenyl, 1-methyl-2-cyclohexenyl, 2-methyl-2-cyclohexenyl, 3-methyl-2-cyclohexenyl, 4-methyl-2-cyclohexenyl, 5-methyl-2-cyclohexenyl, 6-methyl-2-cyclohexenyl, 1-methyl-3-cyclohexenyl, 2-methyl-3-cyclohexenyl, 3-methyl-3-cyclohexenyl, 4-methyl-3-cyclohexenyl, 5-methyl-3-cyclohexenyl, 6-methyl-3-cyclohexenyl, 1-cycloheptenyl, 2-cycloheptenyl, 3-cycloheptenyl, 4-cycloheptenyl, (1'-cyclohexenyl)methyl, (2'-cyclohexenyl)methyl, (3'-cyclohexenyl)methyl, (1',3'-cyclohexadienyl)methyl, (1',4'-cyclohexadienyl)methyl, (1',5'-cyclohexadienyl)methyl, (2',4'-cyclohexadienyl)methyl, and (2',5'-cyclohexadienyl)methyl groups.

As mentioned above, the cycloalkyl groups having 3 to 7 carbon atoms or the cycloalkenyl groups having 3 to 7 carbon atoms may have a hydroxyl group, an alkyloxy group having 1 to 3 carbon atoms, an allyloxy group, or an alkyl group having 1 to 3 carbon atoms as a substituent. Examples of the alkyloxy group having 1 to 3 carbon atoms and the alkyl group having 1 to 3 carbon atoms are as defined above.
Among these cycloalkyl groups having 3 to 7 carbon atoms or the cycloalkenyl groups having 3 to 7 carbon atoms, a cyclohexyl group and a cyclopentyl group are preferable because the effects of the present invention are good.

In R₁ to R₄, as mentioned above, the phenyl group or benzyl group may have a hydroxyl group, an alkyloxy group having 1 to 3 carbon atoms, an allyloxy group, or an alkyl group having 1 to 3 carbon atoms as a substituent. Examples of the alkyloxy group having 1 to 3 carbon atoms and the alkyl group having 1 to 3 carbon atoms are as defined above.
Specifically, examples thereof include 4-methoxyphenyl, 3-methoxyphenyl, 2-methoxyphenyl, 4-ethoxyphenyl, 3-ethoxyphenyl, 2-ethoxyphenyl, 4-propoxyphenyl, 3-propoxyphenyl, 2-propoxyphenyl, 4-allyloxyphenyl, 3-allyloxyphenyl, 2-allyloxyphenyl, 2,4-dimethoxyphenyl, 3,4-dimethoxyphenyl, 3,4,5-trimethoxyphenyl, 2-hydroxyphenyl, 3-hydroxyphenyl, 4-hydroxyphenyl, 2,4-dihydroxyphenyl, 3,4-dihydroxyphenyl, 3,4,5-trihydroxyphenyl, 2-hydroxy-4-methoxyphenyl, 3-hydroxy-4-methoxyphenyl, 4-hydroxy-3-methoxyphenyl, 2-methylphenyl, 3-methylphenyl, 4-methylphenyl, 2-ethylphenyl, 3-ethylphenyl, 4-ethylphenyl, 2-propylphenyl, 3-propylphenyl, 4-propylphenyl,

4-methoxybenzyl, 3-methoxybenzyl, 2-methoxybenzyl, 4-ethoxybenzyl, 3-ethoxybenzyl, 2-ethoxybenzyl, 4-propoxybenzyl, 3-propoxybenzyl, 2-propoxybenzyl, 4-allyloxybenzyl, 3-allyloxybenzyl, 2-allyloxybenzyl, 2,4-dimethoxybenzyl, 3,4-dimethoxybenzyl, 3,4,5-trimethoxybenzyl, 2-hydroxybenzyl, 3-hydroxybenzyl, 4-hydroxybenzyl, 2,4-dihydroxybenzyl, 3,4-dihydroxybenzyl, 3,4,5-trihydroxybenzyl, 2-hydroxy-4-methoxybenzyl, 3-hydroxy-4-methoxybenzyl, 4-hydroxy-3-methoxybenzyl, 2-methylbenzyl, 3-methylbenzyl, 4-methylbenzyl, 2-ethylbenzyl, 3-ethylbenzyl, 4-ethylbenzyl, 2-propylbenzyl, 3-propylbenzyl, and 4-propylbenzyl groups.
Among these, a phenyl group or a benzyl group is preferable because the effects of the present invention and the solubility are good.

In R₁ to R₄, it is preferred that at least one of R₁ and R₂, and/or at least one of R₃ and R₄, each independently represents a hydrogen atom, an alkyl group having 1 to 3 carbon atoms, a cycloalkyl group having 3 to 7 carbon atoms, or a benzyl group because the effects of the present invention and the solubility are good. Particularly preferably, at least one of R₁ and R₂, and/or at least one of R₃ and R₄, each independently represents a hydrogen atom, a methyl group, or an ethyl group.

It is also preferred that R₁ and R₂, and/or R₃ and R₄, independently form a heterocycle having 6 carbon atoms or less in total together with the nitrogen atom to which they are bonded. The heterocycle may have a unsaturated bond, and also may contain an oxygen atom.
Specific examples of such NR₁R₂ or NR₃R₄ include piperidine, pyrrolidine, pyrrole and morpholine. As mentioned above, these heterocycles may have a hydroxyl group, an alkyloxy group having 1 to 3 carbon atoms, an allyloxy group, or an alkyl group having 1 to 3 carbon atoms as a substituent.

Specifically, preferable examples of NR₁R₂ or NR₃R₄ include methylamino, ethylamino, n-propylamino, iso-propylamino, hydroxyethylamino, ethoxyethylamino, dimethylamino, diethylamino, di-n-propylamino, methylethylamino, cyclohexylamino, cyclopentylamino, phenylamino, benzylamino, piperidino, pyrrolidino, pyrrolino, and morpholino groups.

In the present invention, it is preferred that NR₃R₄ is the same as NR₁R₂ because synthesis from glutamic acid is easy. However, the present invention is not limited thereto because the glutamic acid of the present invention in which NR₃R₄ is different from NR₁R₂ is also easily obtainable by using glutamine or theanine as a starting material or using an appropriate synthesis method.

In the glutamic acid derivative of the formula (2), the glutamic acid as a main structure may be any of D-, L-, and DL-forms, and the mixing ratio in DL-form does not be limited. Though the effects of the present invention is good in any forms, the glutamic acid is preferably L-form in terms of the ease of obtaining the glutamic acid derivatives of the present invention.

In the present invention, preferable examples of the glutamic acid derivatives represented by the formula (2) include compounds selected from the group consisting of N-acetyl-N'-methyl-L-glutamic acid-1-amide, N-acetyl-N'-ethyl-L-glutamic acid-1-amide, N-acetyl-N'-n-propyl-L-glutamic acid-1-amide, N-acetyl-N'-benzyl-L-glutamic acid-1-amide, N-acetyl-N'-cyclohexyl-L-glutamic acid-1-amide, N-acetyl-N'-cyclopentyl-L-glutamic acid-1-amide, N-acetyl-L-glutamic acid 1-pyrrolidine amide, N-acetyl-L-glutamic acid 1-piperidine amide, N-acetyl-L-glutamic acid 1-morpholine amide, N-benzoyl-N'-methyl-L-glutamic acid-1-amide, N-benzoyl-N'-ethyl-L-glutamic acid-1-amide, N-benzoyl-N'-n-propyl-L-glutamic acid-1-amide, N-benzoyl-L-glutamic acid 1-morpholine amide, N-acetyl-N',N"-dimethyl-L-glutamic acid-1,5-diamide, N-acetyl-N',N"-diethyl-L-glutamic acid-1,5-diamide, N-acetyl-N',N',N",N"-tetramethyl-L-glutamic acid-1,5-diamide, N-acetyl-N',N',N",N"-tetraethyl-L-glutamic acid-1,5-diamide, N-acetyl-L-glutamic acid bis-1,5-morpholine amide, N-acetyl-L-glutamic acid bis-1,5-piperidine amide, N-acetyl-N'-methyl-DL-glutamic acid-1-amide, N-acetyl-N'-ethyl-DL-glutamic acid-1-amide, N-acetyl-N'-n-propyl-DL-glutamic acid-1-amide, N-acetyl-N'-benzyl-DL-glutamic acid-1-amide, N-acetyl-N'-cyclohexyl-DL-glutamic acid-1-amide, N-acetyl-N'-cyclopentyl-DL-glutamic acid-1-amide, N-acetyl-DL-glutamic acid 1-pyrrolidine amide, N-acetyl-DL-glutamic acid 1-piperidine amide, N-acetyl-DL-glutamic acid 1-morpholine amide, N-benzoyl-N'-methyl-DL-glutamic acid-1-amide, N-benzoyl-N'-ethyl-DL-glutamic acid-1-amide, N-benzoyl-N'-n-propyl-DL-glutamic acid-1-amide, N-benzoyl-DL-glutamic acid 1-morpholine amide, N-acetyl-N',N"-dimethyl-DL-glutamic acid-1,5-diamide, N-acetyl-N',N"-diethyl-DL-glutamic acid-1,5-diamide, N-acetyl-N',N',N",N"-tetramethyl-DL-glutamic acid-1,5-diamide, N-acetyl-N',N',N",N"-tetraethyl-DL-glutamic acid-1,5-diamide, N-acetyl-DL-glutamic acid bis-1,5-morpholine amide, N-acetyl-DL-glutamic acid bis-1,5-piperidine amide, N-acetyl-N'-methyl-D-glutamic acid-1-amide, N-acetyl-N'-ethyl-D-glutamic acid-1-amide, N-acetyl-N'-n-propyl-D-glutamic acid-1-amide, N-acetyl-N'-benzyl-D-glutamic acid-1-amide, N-acetyl-N'-cyclohexyl-D-glutamic acid-1-amide, N-acetyl-N'-cyclopentyl-D-glutamic acid-1-amide, N-acetyl-D-glutamic acid 1-pyrrolidine amide, N-acetyl-D-glutamic acid 1-piperidine amide, N-acetyl-D-glutamic acid 1-morpholine amide, N-benzoyl-N'-methyl-D-glutamic acid-1-amide, N-benzoyl-N'-ethyl-D-glutamic acid-1-amide, N-benzoyl-N'-n-propyl-D-glutamic acid-1-amide, N-benzoyl-D-glutamic acid 1-morpholine amide, N-acetyl-N',N"-dimethyl-D-glutamic acid-1,5-diamide, N-acetyl-N',N"-diethyl-D-glutamic acid-1,5-diamide, N-acetyl-N',N',N",N"-tetramethyl-D-glutamic acid-1,5-diamide, N-acetyl-N',N',N",N"-tetraethyl-D-glutamic acid-1,5-diamide, N-acetyl-D-glutamic acid bis-1,5-morpholine amide, N-acetyl-D-glutamic acid bis-1,5-piperidine amide, and the salts thereof.

Among these, more preferable examples thereof include compounds selected from the group consisting of N-acetyl-N'-methyl-L-glutamic acid-1-amide, N-acetyl-N'-ethyl-L-glutamic acid-1-amide, N-acetyl-N'-n-propyl-L-glutamic acid-1-amide, N-acetyl-N'-benzyl-L-glutamic acid-1-amide, N-acetyl-N'-cyclohexyl-L-glutamic acid-1-amide, N-acetyl-N'-cyclopentyl-L-glutamic acid-1-amide, N-acetyl-L-glutamic acid 1-pyrrolidine amide, N-acetyl-L-glutamic acid 1-piperidine amide, N-acetyl-L-glutamic acid 1-morpholine amide, N-benzoyl-N'-methyl-L-glutamic acid-1-amide, N-benzoyl-N'-ethyl-L-glutamic acid-1-amide, N-benzoyl-N'-n-propyl-L-glutamic acid-1-amide, N-benzoyl-L-glutamic acid 1-morpholine amide, N-acetyl-N',N"-dimethyl-L-glutamic acid-1,5-diamide, N-acetyl-N',N"-diethyl-L-glutamic acid-1,5-diamide, N-acetyl-N',N',N",N"-tetramethyl-L-glutamic acid-1,5-diamide, N-acetyl-N',N',N",N"-tetraethyl-L-glutamic acid-1,5-diamide, N-acetyl-L-glutamic acid bis-1,5-morpholine amide, N-acetyl-L-glutamic acid bis-1,5-piperidine amide, and the salts thereof. These compounds are the most excellent, among the compounds represented by the formula (2), in the effects of inhibiting parakeratosis, shrinking pores, and inhibiting/ameliorating rough skin. In addition, these compounds are favorably soluble in preparations, and have high safety. Thus, these compounds are the most excellent in terms of achieving the objects of the present invention.

The glutamic acid derivatives and the salts thereof according to the present invention can be obtained by the synthesis with use of a known method or the extraction and purification from animals, plants, microorganisms, etc.. The glutamic acid derivatives are also available as commercial products. For example, N-carbamoyl-L-glutamic acid is commercially available from Sigma-Aldrich Corporation, N-benzyloxycarbonyl-L-glutamic acid and N-methyl-DL-glutamic acid are commercially available from Tokyo Chemical Industry Co., Ltd., N-methyl-L-glutamic acid is commercially available from Kokusan Chemical Co., Ltd., and S-2-guanidinoglutaric acid is commercially available from Sigma-Aldrich Corporation.
Representative synthetic examples thereof are presented below, however, the present invention is not limited to these examples.

### (Synthetic Examples)

### (1) N-acetyl-N',N"-dimethyl-L-glutamic acid-1,5-diamide

50 mL of methanol was added to 5 g of N-acetyl-L-glutamic acid, and then 10 mL of 10 % hydrochloric acid-containing methanol solution was added thereto. The mixture was refluxed with heating for one hour, air-cooled, and then concentrated. 20 mL of 40 % methylamine-containing methanol solution was added to the residue, and the mixture was stirred at the room temperature for 2 hours. The reaction mixture was concentrated, dried to be solidified, and then recrystallized with methanol to give 3.8 g of the target compound.
The structure of the obtained target compound was confirmed by an NMR (JEOL EX-400, solvent: DMSO).
¹H-NMR(ppm):8.01(1H), 7.82(1H), 7.73(1H), 4.12(1H), 2.55(6H), 2.05(2H), 1.85(4H), 1.68(1H). ¹³C-NMR(ppm):171.8, 171.8, 169.2, 62.3, 31.8, 28.1, 25.5, 25.4, 22.5.

### (2) N-acetyl-N',N"-diethyl-L-glutamic acid-1,5-diamide

50 mL of methanol was added to 5 g of N-acetyl-L-glutamic acid, and then 50 mL of 10 % hydrochloric acid-containing methanol solution was added thereto. The mixture was refluxed with heating for one hour, air-cooled, and then concentrated. 20 mL of 70 % ethylamine-containing aqueous solution was added to the residue, and the mixture was stirred at 50 °C for 2 hours. The reaction mixture was concentrated, dried to be solidified, and recrystallized with methanol to give 3.7 g of the target compound.

### (3) N-acetyl-N',N',N",N"-tetramethyl-L-glutamic acid-1,5-diamide

50 mL of methanol was added to 5 g ofN-acetyl-L-glutamic acid, and then 50 mL of 10 % hydrochloric acid-containing methanol solution was added thereto. The mixture was refluxed with heating for one hour, air-cooled, and then concentrated. 20 mL of dimethylamine was added to the residue, and the mixture was stirred at 50 °C for 3 hours. The reaction mixture was concentrated and dried to give 3.7 g of the target compound in a liquid state.

### (4) N-acetyl-N'-methyl-L-glutamic acid-1-amide

10 g of N-benzyloxycarbonyl-tert-butyl-L-glutamic acid-5-ester was dissolved in 200 mL of methylene chloride, and then g of methylamine hydrochloride, 4.9 mL of triethylamine, 6.7 g of EDCI, and 5.4 g of HOBt were added thereto at the room temperature. The mixture was stirred at the room temperature overnight, diluted with 50 mL of methylene chloride, washed with 50 mL of water, 50 mL of 2M hydrochloric acid, and 50 mL of saturated sodium bicarbonate aqueous solution, dried with anhydrous magnesium sulfate, and then filtered. The filtrate was concentrated to give 9.1 g of residue. The obtained residue was dissolved in 200 mL of methanol, and then 1 g of 5 % Pd-C (50% wet) was added thereto. The mixture was stirred at the room temperature overnight under hydrogen atmosphere, and then filtered. The filtrate was concentrated to give 5.9 g of residue.

1.02 of the obtained residue was dissolved in 30 mL of methylene chloride, and then 1.4 mL of triethylamine and 525 µl of acetyl chloride were added thereto. The mixture was stirred at the room temperature overnight and then 1 mL of methanol was added thereto. The mixture was stirred for 10 minutes, diluted with 50 mL of methylene chloride, washed with 50 mL of water, 50 mL of 2M hydrochloric acid, 50 mL of saturated sodium bicarbonate aqueous solution, and 50 mL of saturated brine, dried with anhydrous magnesium sulfate, and then filtered. After the filtrate was concentrated, 10 mL of dioxane was added to the residue, and then 10 mL of 4M hydrochloric acid-containing dioxane solution was added thereto at the room temperature. The mixture was stirred at the room temperature overnight, and then the solvent was distilled away under reduced pressure to give 0.4 g of the target compound.

### (5) N-benzoyl-N'-methyl-L-glutamic acid-1-amide

1.02 g of the residue obtained in (4) was dissolved in 30 mL of methylene chloride, and then 1.4 mL of triethylamine and 900 µl of benzoyl chloride were added thereto. The mixture was stirred at the room temperature overnight, and then 1 mL of N,N-dimethylethylenediamine was added thereto. The mixture was stirred for 10 minutes, diluted with 50 mL of methylene chloride, washed with 50 mL of water, 50 mL of 2M hydrochloric acid, 50 mL of saturated sodium bicarbonate aqueous solution, and 50 mL of saturated brine, dried with anhydrous magnesium sulfate, and then filtered. After the filtrate was concentrated, 10 mL of dioxane was added to the residue, and then 10 mL of 4M hydrochloric acid-containing dioxane solution was added thereto at the room temperature. The mixture was stirred at the room temperature overnight, and then the solvent was distilled away under reduced pressure to give 1.4 g of the target compound.

### (6) N-acetyl-N'-ethyl-L-glutamic acid-1-amide

10 g of N-benzyloxycarbonyl-L-glutamic acid-tert-butyl-5-ester was dissolved in 200 mL of methylene chloride, and then 4.9 mL of ethylamine hydrochloride triethylamine, 6.7 g of EDCI, and 5.4 g of HOBt were added thereto at the room temperature. The mixture was stirred at the room temperature overnight, diluted with 50 mL of methylene chloride, washed with 50 mL of water, 50 mL of 2M hydrochloric acid, and 50 mL of saturated sodium bicarbonate aqueous solution, dried with anhydrous magnesium sulfate, and then filtered. The filtrate was concentrated to give 9.4 g of residue. The obtained residue was dissolved in 200 mL of methanol, and 1 g of 5 % Pd-C (50% wet) was added thereto. The mixture was stirred at the room temperature overnight under hydrogen atmosphere, and then filtered. The filtrate was concentrated to give 6.2 g of residue.

1.15 of the obtained residue was dissolved in 30 mL of methylene chloride, and then 1.4 mL of triethylamine and 525 µl of acetyl chloride were added thereto. The mixture was stirred at the room temperature overnight, and then 1 mL of methanol was added thereto. The mixture was stirred for 10 minutes, was diluted with 50 mL of methylene chloride, washed with 50 mL of water, 50 mL of 2M hydrochloric acid, 50 mL of saturated sodium bicarbonate aqueous solution, and 50 mL of saturated brine, dried with anhydrous magnesium sulfate, and then filtered. After the filtrate was concentrated, 10 mL of dioxane was added to the residue, and then 10 mL of 4M hydrochloric acid-containing dioxane solution was added thereto at the room temperature. The mixture was stirred at the room temperature overnight, and then the solvent was distilled away under reduced pressure to give 0.6 g of the target compound.

### (7) N-benzoyl-N'-ethyl-L-glutamic acid-1-amide

1.15 g of the residue obtained in (6) was dissolved in 30 mL of methylene chloride, and then 1.4 mL of triethylamine and 900 µl of benzoyl chloride were added thereto. The mixture was stirred at the room temperature overnight, and then 1 mL of N,N-dimethylethylenediamine was added thereto. The mixture was stirred for 10 minutes, diluted with 50 mL of methylene chloride, washed with 50 mL of water, 50 mL of 2M hydrochloric acid, 50 mL of saturated sodium bicarbonate aqueous solution, and 50 mL of saturated brine, dried with anhydrous magnesium sulfate, and then filtered. After the filtrate was concentrated, 10 mL of dioxane was added to the residue, and then 10 mL of 4M hydrochloric acid-containing dioxane solution was added thereto at the room temperature. The mixture was stirred at the room temperature overnight, and then the solvent was distilled away under reduced pressure to give 1.6 g of the target compound.

### (8) N-acetyl-N'-benzyl-L-glutamic acid-1-amide

10 g of N-benzyloxycarbonyl-L-glutamic acid-tert-butyl-5-ester was dissolved in 200 mL of methylene chloride, and then 3.75 g of benzylamine, 6.7 g of EDCI, and 5.4 g of HOBt were added thereto at the room temperature. The mixture was stirred at the room temperature overnight, diluted with 50 mL of methylene chloride, washed with 50 mL of water, 50 mL of 2M hydrochloric acid, and 50 mL of saturated sodium bicarbonate aqueous solution, dried with anhydrous magnesium sulfate, and then filtered. After the filtrate was concentrated, the obtained residue was dissolved in 200 mL of methanol, and 1 g of 5 % Pd-C (50% wet) was added thereto. The mixture was stirred at the room temperature overnight under hydrogen atmosphere, and then filtered. The filtrate was concentrated to give 7.8 g of residue.

1.46 g of the obtained residue was dissolved in 30 mL of methylene chloride, and then 1.4 mL of triethylamine and 525 µl of acetyl chloride were added thereto. The mixture was stirred for 10 minutes, diluted with 50 mL of methylene chloride, washed with 50 mL of water, 50 mL of 2M hydrochloric acid, 50 mL of saturated sodium bicarbonate aqueous solution, and 50 mL of saturated brine, dried with anhydrous magnesium sulfate, and then filtered. After the filtrate was concentrated, 10 mL of dioxane was added to the residue, and then 10 mL of 4M hydrochloric acid-containing dioxane solution was added thereto at the room temperature. The mixture was stirred at the room temperature overnight, and then the solvent was distilled away under reduced pressure to give 1.3 g of the target compound.

### (9) N-benzoyl-N'-benzyl-L-glutamic acid-1-amide

1.46 g of the residue obtained in (8) was dissolved in 30 mL of methylene chloride, and then 1.4 mL of triethylamine and 900 µl of benzoyl chloride were added thereto. The mixture was stirred at the room temperature overnight, and then 1 mL of N,N-dimethylethylenediamine was added thereto. The mixture was stirred for 10 minutes, diluted with 50 mL of methylene chloride, washed with 50 mL of water, 50 mL of 2M hydrochloric acid, 50 mL of saturated sodium bicarbonate aqueous solution, and 50 mL of saturated brine, dried with anhydrous magnesium sulfate, and then filtered. After the filtrate was concentrated, 10 mL of dioxane was added to the residue, and then 10 mL of 4M hydrochloric acid-containing dioxane solution was added thereto at the room temperature. The mixture was stirred at the room temperature overnight, and then the solvent was distilled away under reduced pressure to give 1.3 g of the target compound.

The glutamic acid derivative of the present invention may be used as a salt, however, it is not always necessary. The kinds of salts are not limited in particular as long as they are pharmacologically or pharmaceutically acceptable salts. Examples of inorganic salts include sodium salt, potassium salt, calcium salt, zinc salt, magnesium salt, ammonium salt, hydrochloride, hydrosulfate, phosphate, and hydrobromate. Examples of organic salts include methylamine salt, pyridine salt, trimethylamine salt, triethanolamine salt, methylsulfate, p-toluenesulfonate, acetate, lactate, maleate, fumarate, oxalate, succinate, tartrate, citrate, betaine salt, glycine salt, sarine salt, and taurine salt, and the salts of the present invention are not limited to the above-mentioned salts. The salts can be obtained by a known method.

In the process of skin metabolism, the keratinocyte moving to the uppermost layer of skin (skin surface) will eventually slough off as scurf. The keratinocyte in such a state usually loses the nucleus. However, when for any cause, the differentiation or proliferation of skin cells are disordered and the speed of keratinization is unusually quickened, epidermal keratinocytes in an immature state that they have nuclei inside themselves are sometimes present in the stratum corneum. This state is referred to as parakeratosis. In the present invention, parakeratosis of the epidermal keratinocytes is also described simply as "parakeratosis". The term described simply as "parakeratosis" means parakeratosis of epidermal keratinocytes.
An enlarged pore becoming conspicuous is funnel-shaped, and the stratum corneum of funnel-shaped area surrounding a follicle of the pore is in a state of parakeratosis. Thus, when parakeratosis occurs in the stratum corneum surrounding a follicle(where a hair vents itself) of the pore, the area surrounding the follicle becomes funnel-shaped. Such an enlarged funnel-shaped structure of the pore make the pore conspicuous.

The glutamic acid derivatives and the salts thereof according to the present invention (hereinafter, they are also referred to as glutamic acid derivatives) have excellent effects of inhibiting parakeratosis and shrinking pores as demonstrated below. Moreover, they also have the effect of inhibiting/ameliorating rough skin. Thus, one or more compounds selected from the group consisting of the glutamic acid derivatives according to the present invention are useful as a parakeratosis inhibitor, a pore-shrinking agent, and a rough skin inhibiting/ameliorating agent, and they are preferably incorporated in an external composition for skin, particularly an external composition for skin for inhibiting parakeratosis, shrinking pores, or inhibiting/ameliorating rough skin.

These are novel and useful applications of the glutamic acid derivatives according to the present invention based on the discovery of the above-mentioned novel effects thereof.
It has ever been not known that the glutamic acid derivatives represented by the formula (1a) or (2) according to the present invention, the salts thereof, and the external composition for skin containing the same have the effects of inhibiting parakeratosis, shrinking pores, and inhibiting/ameliorating rough skin, and it has been first found by the present inventors.
In addition, it has ever been not known that N-amidinoglutamic acid represented by the structural formula (1b) according to the present invention, the salt thereof, and the external composition for skin containing the same have the effects of inhibiting parakeratosis and shrinking pores, and it has been first found by the present inventors.

The parakeratosis inhibitor, the pore-shrinking agent, the rough skin inhibiting/ameliorating agent, and the external composition for skin have a very wide application range and can be used in a variety of fields such as cosmetics including quasi-drugs, pharmaceutical products, and food products.

The composition containing one or more compounds selected from the group consisting of the glutamic acid derivatives is useful as a composition for inhibiting parakeratosis, shrinking pores, or inhibiting or ameliorating rough skin, and the compositions have excellent effects of inhibiting parakeratosis, shrinking pores, and inhibiting/ameliorating rough skin.

Examples of the external composition for skin include basic cosmetics such as face washes, milky lotions, creams, lotions, gels, essences (beauty essences), packs, and masks; makeup cosmetics such as foundations and lip rouges; oral cosmetics such as dentifrices; cosmetics such as fragrances, hair cosmetics, and body cosmetics; and ointments.
When used in the face, the external composition for skin according to the present invention ameliorates conspicuous pores in the nose, cheeks, and so on as well as inhibits/ameliorates rough skin. When used in the body such as legs after hair removal, it ameliorates conspicuous pores as well as inhibits/ameliorates rough skin. In addition, it can provide skin with a youthful and fresh appearance without conspicuous pores by inhibiting parakeratosis, maintaining or improving the skin to a healthy state, and further shrinking pores.

It is possible to prepare the composition of the present invention by using only the one or more compounds selected from the group consisting of the glutamic acid derivatives. However, the amount of the one or more compounds selected from the group consisting of the glutamic acid derivatives is preferably 0.01 to 3.0 mass%, and more preferably 0.05 to 1.0 mass%, with respect to the total amount of the composition.

The composition according to the present invention can take product forms such as solutions (e.g., aqueous solutions, alcoholic aqueous solutions, and oil solutions), milky lotions, creams, gels, jellies, suspensions, capsules, micro capsules, solids, powder, and granules. It can take systems such as solutions(e.g., aqueous solutions, alcoholic aqueous solutions, and oil solutions), solubilized systems, emulsions, gels, jellies, dispersions, aerosols, water-oil two-phase systems, and water-oil-powder three-phase systems.

The composition according to the present invention can be prepared by a conventional method. In the composition, in addition to one or more compounds selected from the group consisting of the glutamic acid derivatives, other components generally used in cosmetics including quasi drugs, pharmaceuticals, foods, and so on can be incorporated appropriately, as necessary. Examples of such other components include oils, surfactants, powders, coloring agents, water, alcohols, thickening agents, chelating agents, silicones, antioxidants, UV absorbers, moisturizers, perfumes, various kinds of drug components, preservatives, pH adjusters, and neutralizers.

In the arbitrarily-incorporated components which are incorporated appropriately as mentioned above, examples of the oils include: higher alcohols such as linear alcohols (e.g., lauryl alcohol, cetyl alcohol, stearyl alcohol, myristyl alcohol, and oleyl alcohol), and branched-chain alcohols (e.g., monostearyl glycerin ether, lanolin alcohol, cholesterol, phytosterol, and isostearyl alcohol); higher fatty acids such as lauric acid, myristic acid, palmitic acid, and stearic acid; waxes such as solid paraffin, beeswax, hydrogenated castor oil, carnauba wax, and bareco wax; vegetable or animal fats and oils such as beef tallow, pork tallow, mutton tallow, squalane, coconut oil, palm oil, palm kernel oil, soybean oil, olive oil, cottonseed oil, jojoba oil, castor oil, and lanoline; mineral oils such as liquid paraffin and petrolatum; and synthetic oils such as trimethylpropane triisostearate, isopropyl myristate, glyceryl tri-2-ethylhexanoate, pentaerythritol tetra-2-ethylhexanoate, silicone oil, polyoxyethylene (hereinafter it is also referred to as POE)-polyoxypropylene (hereinafter it is also referred to as POP)-pentaerythritol ether.

Examples of the surfactants include anionic surfactants such as fatty acid soaps (e.g., soap base, sodium laurate, and sodium palmitate), higher alkyl sulfate salts (e.g., sodium lauryl sulfate and potassium lauryl sulfate), alkyl ether sulfate salts (e.g., triethanolamine POE lauryl sulfate and sodium POE lauryl sulfate), N-acylsarcosinates (e.g., sodium lauroylsarcosinate), higher fatty acid amide sulfonate salts (e.g., sodium N-myristoyl-N-methyltaurate and sodium cocoyl methyltaurare), phosphate ester salts (e.g., POE stearyl ether phosphate), sulfosuccinates (e.g., sodium monolauroylmonoethanolamide POE sulfosuccinate and sodium laurylpolypropylene glycol sulfosuccinate), alkylbenzenesulfonates (e.g., sodium linear dodecylbenzenesulfonate and linear triethanolamine dodecylbenzenesulfonate), N-acylglutamic acid salts (e.g., disodium N-stearoylglutamate and monosodium N-stearoylglutamate), higher fatty acid ester sulfate salts (e.g., sodium hydrogenatad cocoylglyceride sulfate), sulfated oils (e.g., Turkey red oil), POE alkyl ether carboxylic acid, POE alkyl allyl ether carboxylic acid salt, higher fatty acid ester sulfonate, secondary alcohol sulfate salt, higher fatty acid alkylolamide sulfate salt, sodium lauroyl monoethanolamide succinate, and sodium caseinate;

cationic surfactants such as alkyltrimethylammonium salts (e.g., stearyltrimethylammonium chloride and lauryltrimethylammonium chloride), dialkyldimethylammonium salts (e.g., distearyldimethylammonium chloride), alkylpyridinium salts (e.g., cetylpyridinium chloride), quaternary alkylammonium salt, alkyldimethylbenzylammonium salt, alkylisoquinolinium salt, dialkylmorpholinium salt, POE alkylamine, alkylamine salt, polyamine fatty acid derivatives, amyl alcohol fatty acid derivatives, and benzalkonium chloride; amphoteric surfactants such as imidazoline-based amphoteric surfactants (e.g., 2-cocoyl-2-imidazoliniumhydroxide-1-carboxyethyloxy disodium salt), and betaine-based surfactants (e.g., amidobetaine and sulfobetaine); lipophilic nonionic surfactants such as sorbitan fatty acid esters (e.g., sorbitan monooleate, sorbitan monoisostearate, sorbitan monolaurate, sorbitan monopalmitate, and sorbitan trioleate), glycerin or polyglycerin fatty acids (e.g., cotton oil fatty acid monoglyceride, glyceryl monostearate, glyceryl sesquioleate, and glyceryl monostearate malate), propylene glycol fatty acid esters (e.g., propylene glycol monostearate), hydrogenated castor oil derivatives, glycerin alkyl ethers, and POE-methylpolysiloxane copolymer; hydrophilic nonionic surfactants such as POE sorbitan fatty acid esters (e.g., POE sorbitan monooleate and POE sorbitan monostearate), POE sorbitol fatty acid esters (e.g., POE sorbitol monolaurate, POE sorbitol monooleate, and POE sorbitol monostearate), POE glycerin fatty acid esters (e.g., POE glycerin monooleate and POE glycerin distearate), POE fatty acid esters (e.g., POE monooleate, POE distearate, and POE monodioleate), POE alkyl ethers (e.g., POE lauryl ether, POE oleyl ether, and POE cholestanol ester), POE alkyl phenyl ethers (e.g., POE octyl phenyl ether and POE nonyl phenyl ether), Pluronic types (e.g., Pluronic), POE-POP alkyl ethers (e.g., POE-POP monobutyl ether, POE-POP cetyl ether, and POE-POP glycerin ether), POE castor oil or hydrogenated castor oil derivatives (e.g., POE castor oil, POE hydrogenated castor oil, POE hydrogenated castor oil monoisostearate, and POE hydrogenated castor oil maleate), POE beeswax or lanolin derivatives (e.g., POE sorbit beeswax), alkanol amides (e.g., cocoyldiethanolamide and fatty acid isopropanolamide), POE propylene glycol fatty acid esters, POE fatty acid amides, POE alkylamines, sucrose fatty acid esters, and alkylethoxydimethylamine oxides.

Examples of the alcohol include lower alcohols such as ethanol, propanol, and isopropanol.

Examples of the thickener include water-soluble polymers such as plant-derived polymers (e.g., gum arabic, tragacanth gum, galactan, carob gum, guar gum, carrageenan, pectin, agar, and starch (corn, wheat, potato, and rice)), microorganism-derived polymers (e.g., dextran and pullulan), starch polymers (e.g., carboxymethyl starch and methylhydroxypropyl starch), animal-derived polymers (e.g., collagen, casein, and gelatin), cellulose polymers (e.g., methylcellulose, nitrocellulose, ethylcellulose, hydroxyethylcellulose, sodium cellulose sulfate, hydroxypropylcellulose, carboxymethylcellulose, and crystalline cellulose), alginate polymers (e.g., sodium alginate and propylene glycol alginate), vinyl polymers (e.g., polyvinyl methyl ether and carboxyvinyl polymer); POE-based polymers, POE-POP copolymer-based polymers, acrylic polymers (e.g., sodium polyacrylate and polyacrylamide), polyethyleneimine, cationic polymer, and inorganic water-soluble polymers (e.g., bentonite, magnesium aluminium silicate, laponite, hectorite, and silicic anhydride).

Examples of the chelating agent include citramalic acid, agaric acid, glyceric acid, shikimic acid, hinokitiol, gallic acid, tannic acid, caffeic acid, ethylenediaminetetraacetic acid, ethyleneglycoldiaminetetraacetic acid, diethylenetriaminepentaacetic acid, phytic acid, polyphosphoric acid, metaphosphoric acid, the analogs thereof, the alkali metal salts thereof, and carboxylic acid ester.

Examples of the UV absorber include benzoic acid-based UV absorbers such as p-aminobenzoic acid; anthranilic acid-based UV absorbers such as methyl anthranilate; salicylic acid-based UV absorbers such as octyl salicylate; and cinnamic acid-based UV absorbers such as isopropyl p-methoxycinnamate and octyl p-methoxycinnamate.

Examples of the moisturizer include polyethylene glycol, propylene glycol, dipropylene glycol, 1,3-butylene glycol, glycerin, diglycerin, xylitol, maltitol, maltose, D-mannite, glucose, fructose, sodium chondroitin sulfate, sodium hyaluronate, sodium lactate, glucosamine, and cyclodextrin.

As drug components, vitamins such as vitamin A oil, retinol, retinol palmitate, pyridoxine hydrochloride, benzyl nicotinate, nicotinamide, dl-α-tocopherol nicotinate, magnesium ascorbyl phosphate, vitamin D2, dl-α-tocopherol, pantothenic acid, and biotin; anti-inflammatory agents such as azulene and glycyrrhizinic acid; whitening agents such as arbutin, potassium 4-methoxysalicylate, 2-O-ethylascorbic acid, and ascorbic acid glucoside; hormones such as estradiol; astringent agents such as zinc oxide and tannic acid; refresheners such as L-menthol and camphor; and others such as lysozyme chloride, pyridoxine hydrochloride, and sulfur can be incorporated. Moreover, various extracts which provide drug efficacy can be incorporated. Examples of such extracts include houttuynia cordata extract, pPhellodendron extract, glycyrrhiza glabra (licorice) extract, paeonia albiflora root extract, paeonia suffruticosa root extract, luffa cylindrical extract, saxifraga sarmentosa extract, eucalyptus globulus leaf extract, eugenia caryophyllus (clove) flower extract, aesculus hippocastanum (horse chestnut) extract, centaurea cyanus flower extract, seaweed extract, and thymus vulgaris (thyme) extract.

Examples of the antiseptic agent include paraoxybenzoates such as methylparaben, ethylparaben, and butylparaben; benzoic acid; salicylic acid; sorbic acid; p-chloro-m-cresol; hexachlorophene; benzalkonium chloride; chlorhexidine hydrochloride; trichlorocarbanilide; photosensitizer; phenoxyethanol; and isomethylthiazolinone.

Examples of the neutralizer include 2-amino-2-methyl-1-propanol, 2-amino-2-methyl-1,3-propanediol, potassium hydroxide, triethanolamine, and sodium carbonate.
Examples of the pH adjuster include lactic acid, citric acid, glycolic acid, succinic acid, tartaric acid, malic acid, sodium hydrogencarbonate, and ammonium hydrogencarbonate.

Examples of the antioxidant include ascorbic acid, α-tocopherol, and carotenoid.
The above-mentioned components are exemplary, and the present invention is not limited to these examples. These components can be incorporated in any combination in accordance with formulations depending on the desired forms.

### EXAMPLES

Hereinafter, the present invention will be more specifically described by examples: however, the technical scope of the present invention is not limited thereto. The blending quantity is expressed in mass% unless otherwise noted.

### Test Example 1 Test for parakeratosis-inhibiting effect

3 mass% test compound aqueous solution (containing 30 mass% ethanol) was primarily prepared as a sample solution. The pH was adjusted with hydrochloric acid or sodium hydroxide so as to be 7.0 to 7.5. When the solubility of test compound was low, the sample solution was prepared depending on the solubility.

100 µl of 10 mass% oleic acid solution (solvent: ethanol) was applied on the back of a hairless mouse (HR-1; Hoshino Laboratory Animals), and then 100 µl of the sample solution was applied thereon. The day after this procedure was continued for 3 days, the condition of the back skin was observed with a CCD camera, and the rough skin condition (desquamation and erythema) was evaluated. In the evaluation, the condition of the skin on which the control (control aqueous solution) was applied set a score of 2.0 and the skin condition without roughness set a score of 0.0. Then, the skin condition was scored by visual observation in increments of 0.25 between them. At the same time, the stratum corneum at the back of the hairless mouse was peeled off with tape, the nuclei were stained by hematoxylin, and the degree of parakeratosis was observed and evaluated using a parakeratosis value within a range of 1.0 to 3.0 (in 0.25 increments). The higher the value is, the more the number of nucleated cell in the stratum corneum is, i.e., the more the parakeratosis is advanced.
The evaluation was conducted according to the plan which was approved in the in-house council on animal experiment, complying with Act on Welfare and Management of Animals and other related acts.

**Table 1**

| Sample | Concentration (mass%) | Visual observation value(Average for 4 mice) | Parakeratosis value(Average for 4 mice) |
|---|---|---|---|
| Control aqueous solution | | 2.0 | 2.0 |
| N-carbamoyl-L-glutamic acid | 3 | 0.7 | 0.7 |
| N-benzyloxycarbonyl-L-glutamic acid | 3 | 0.9 | 0.9 |
| N-methyl-L-glutamic acid | 3 | 0.8 | 0.8 |
| N-methyl-DL-glutamic acid | 3 | 0.9 | 0.9 |
| N-acetyl-N'-methyl-L-glutamic acid-1-amide | 3 | 0.9 | 0.9 |
| N-acetyl-N'-ethyl-L-glutamic acid-1-amide | 3 | 1.0 | 0.9 |
| N-acetyl-N'-n-propyl-L-glutamic acid -1-amide | 3 | 0.8 | 0.8 |
| N-acetyl-N'-benzyl-L-glutamic acid-1-amide | 3 | 0.8 | 0.8 |
| N-acetyl-N'-cyclohexyl-L-glutamic acid-1-amide | 3 | 0.7 | 0.7 |
| N-acetyl-N'-cyclopentyl-L-glutamic acid-1-amide | 3 | 0.7 | 0.6 |
| N-acetyl-L-glutamic acid 1-pyrrolidine amide | 3 | 0.8 | 0.8 |
| N-acetyl-L-glutamic acid 1-piperidine amide | 3 | 0.7 | 0.7 |
| N-acetyl-L-glutamic acid 1-morpholine amide | 3 | 0.8 | 0.8 |
| N-benzoyl-N'-methyl-L-glutamic acid -1-amide | 3 | 0.6 | 0.5 |
| N-benzoyl-N'-ethyl-L-glutamic acid-1-amide | 3 | 0.7 | 0.7 |
| N-benzoyl-N'-n-propyl-L-glutamic acid -1-amide | 3 | 0.6 | 0.6 |
| N-benzoyl-L-glutamic acid 1-morpholine amide | 3 | 0.7 | 0.7 |
| N-acetyl-N',N"-dimethyl-L-glutamic acid -1,5-diamide | 3 | 0.7 | 0.7 |
| N-acetyl-N',N"-diethyl-L-glutamic acid -1,5-diamide | 3 | 0.8 | 0.8 |
| N-acetyl-N',N',N",N"-tetramethyl-L-glutamic acid-1,5-diamide | 3 | 0.8 | 0.8 |
| N-acetyl-N',N",N",N"-tetraethyl-L-glutamic acid-1,5-diamide | 3 | 0.9 | 0.9 |
| N-acetyl-L-glutamic acid bis-1,5-morpholine amide | 3 | 1.0 | 1.0 |
| N-acetyl-L-glutamic acid bis-1,5-piperidine amide | 3 | 0.9 | 0.9 |
| N-acetyl-L-glutamine (comparative example) | 3 | 1.9 | 1.9 |
| N-acetyl-L-glutamic acid (comparative example) | 3 | 1.2 | 1.2 |

As is clear from Table 1, the glutamic acid derivatives according to the present invention, such as N-carbamoyl-L-glutamic acid, N-benzyloxycarbonyl-L-glutamic acid, N-methyl-L-glutamic acid, N-methyl-DL-glutamic acid, N-acetyl-N'-methyl-L-glutamic acid-1-amide, N-acetyl-N'-ethyl-L-glutamic acid-1-amide, N-acetyl-N'-n-propyl-L-glutamic acid-1-amide, N-acetyl-N'-benzyl-L-glutamic acid-1-amide, N-acetyl-N'-cyclohexyl-L-glutamic acid-1-amide, N-acetyl-N'-cyclopentyl-L-glutamic acid-1-amide, N-acetyl-L-glutamic acid 1-pyrrolidine amide, N-acetyl-L-glutamic acid 1-piperidine amide, N-acetyl-L-glutamic acid 1-morpholine amide, N-benzoyl-N'-methyl-L-glutamic acid-1-amide, N-benzoyl-N'-ethyl-L-glutamic acid-1-amide, N-benzoyl-N'-n-propyl-L-glutamic acid-1-amide, N-benzoyl-L-glutamic acid 1-morpholine amide, N-acetyl-N',N"-dimethyl-L-glutamic acid-1,5-diamide, N-acetyl-N',N"-diethyl-L-glutamic acid-1,5-diamide, N-acetyl-N',N',N",N"-tetramethyl-L-glutamic acid-1,5-diamide, N-acetyl-N',N',N",N"-tetraethyl-L-glutamic acid-1,5-diamide, N-acetyl-L-glutamic acid bis-1,5-morpholine amide, and N-acetyl-L-glutamic acid bis-1,5-piperidine amide, showed the effects of inhibiting rough skin such as desquamation and erythema caused by unsaturated fatty acids.

Also, the glutamic acid derivatives according to the present invention lowered the parakeratosis value. Thus, the effect of inhibiting parakeratosis was confirmed. Inhibition of parakeratosis is effective in shrinking pores.
On the other hand, N-acetyl glutamine as a comparative example did not show such effects, and the effects of N-acetyl glutamic acid were inferior to those of the compounds of the present invention.

**Table 2**

| Sample | Concentration (mass%) | Parakeratosis value (Average for 5 mice) |
|---|---|---|
| Control aqueous solution | - | 2.0 |
| N-amidino-L-glutamic acid | 3.0 | 1.1 |
| N-amidino-D-glutamic acid | 3.0 | 1.2 |
| N-amidino-DL-glutamic acid | 3.0 | 1.2 |
| Sodium N-amidino-L-glutamate | 3.0 | 1.1 |
| N-amidino-L-glutamic acid hydrochloride | 3.0 | 1.1 |
| Citrulline (comparative example) | 3.0 | 2.0 |
| N-acetyl-L-glutamine (comparative example) | 3.0 | 1.9 |

As is clear from Table 2, the N-amidinoglutamic acids according to the present invention, such as N-amidino-L-glutamic acid, N-amidino-D-glutamic acid, N-amidino-DL-glutamic acid, sodium N-amidino-L-glutamate, and N-amidino-L-glutamic acid hydrochloride, lowered the parakeratosis value. Thus, the effect of inhibiting parakeratosis caused by unsaturated fatty acids was confirmed. As mentioned before, inhibition of parakeratosis is effective in shrinking pores.
On the other hand, N-acetyl glutamine and citrulline, which an example of the NOS inhibitors described in Patent Literatures 3 and 4, as comparative examples did not show such effects.

### Test Example 2 Test for ameliorating effect to rough skin induced by applying oleic acid

In order to further investigate the ameliorating effect to the rough skin induced by oleic acid application, the transepidermal water loss (TEWL value) before and after the application was measured. The difference was compared with the control (control aqueous solution), and the effect was determined. The method for preparing and applying the sample was in accordance with Test Example 1. The TEWL was measured using TEWA Meter TM210 (Courage + Khazaka Electronic GmBH).

100 µl of 10 mass% oleic acid solution (solvent: ethanol) was applied on the back of hairless mice (HR-1, four mice per group), and then 100 µl of the sample solution was applied thereon. The day after this procedure was continued for 3 days, the TEWL value of the back was measured, and the average value of the four mice was calculated. The results are shown in Table 3. The higher the TEWL value is, the worse rough skin becomes.

**Table 3**

| Sample | Concentration (mass%) | TEWL value |
|---|---|---|
| Control aqueous solution | - | 12.0 |
| N-carbamoyl-L-glutamic acid | 3 | 6.4 |
| N-benzyloxycarbonyl-L-glutamic acid | 3 | 6.8 |
| N-methyl-L-glutamic acid | 3 | 6.4 |
| N-methyl-DL-glutamic acid | 3 | 7.9 |
| N-acetyl-N'-methyl-L-glutamic acid-1-amide | 3 | 6.6 |
| N-acetyl-N'-ethyl-L-glutamic acid-1-amide | 3 | 6.2 |
| N-acetyl-N'-n-propyl-L-glutamic acid-1-amide | 3 | 7.4 |
| N-acetyl-N'-benzyl-L-glutamic acid-1-amide | 3 | 7.9 |
| N-acetyl-N'-cyclohexyl-L-glutamic acid-1-amide | 3 | 8.5 |
| N-acetyl-N'-cyclopentyl-L-glutamic acid-1-amide | 3 | 8.4 |
| N-acetyl-L-glutamic acid 1-pyrrolidine amide | 3 | 8.0 |
| N-acetyl-L-glutamic acid 1-piperidine amide | 3 | 6.9 |
| N-acetyl-L-glutamic acid 1-morpholine amide | 3 | 6.3 |
| N-benzoyl-N'-methyl-L-glutamic acid-1-amide | 3 | 6.2 |
| N-benzoyl-N'-ethyl-L-glutamic acid-1-amide | 3 | 6.9 |
| N-benzoyl-N'-n-propyl-L-glutamic acid-1-amide | 3 | 7.2 |
| N-benzoyl-L-glutamic acid 1-morpholine amide | 3 | 6.4 |
| N-acetyl-N',N"-dimethyl-L-glutamic acid-1,5-diamide | 3 | 6.3 |
| N-acetyl-N',N"-diethyl-L-glutamic acid-1,5-diamide | 3 | 7.4 |
| N-acetyl-N",N',N",N"-tetramethyl-L-glutamic acid-1,5-diamide | 3 | 7.5 |
| N-acetyl-N',N',N",N"-tetraethyl-L-glutamic acid-1,5-diamide | 3 | 7.6 |
| N-acetyl-L-glutamic acid bis-1,5-morpholine amide | 3 | 7.9 |
| N-acetyl-L-glutamic acid bis-1,5-piperidine amide | 3 | 6.9 |
| N-acetyl-L-glutamine (comparative example) | 3 | 13.2 |
| N-acetyl-L-glutamic acid (comparative example) | 3 | 10.1 |

As is clear from Table 3, the application of the glutamic acid derivatives according to the present invention, such as N-carbamoyl-L-glutamic acid, N-benzyloxycarbonyl-L-glutamic acid, N-methyl-L-glutamic acid, N-methyl-DL-glutamic acid, N-acetyl-N'-methyl-L-glutamic acid-1-amide, N-acetyl-N'-ethyl-L-glutamic acid-1-amide, N-acetyl-N'-n-propyl-L-glutamic acid-1-amide, N-acetyl-N'-benzyl-L-glutamic acid-1-amide, N-acetyl-N'-cyclohexyl-L-glutamic acid-1-amide, N-acetyl-N'-cyclopentyl-L-glutamic acid-1-amide, N-acetyl-L-glutamic acid 1-pyrrolidine amide, N-acetyl-L-glutamic acid 1-piperidine amide, N-acetyl-L-glutamic acid 1-morpholine amide, N-benzoyl-N'-methyl-L-glutamic acid-1-amide, N-benzoyl-N'-ethyl-L-glutamic acid-1-amide, N-benzoyl-N'-n-propyl-L-glutamic acid-1-amide, N-benzoyl-L-glutamic acid 1-morpholine amide, N-acetyl-N',N"-dimethyl-L-glutamic acid-1,5-diamide, N-acetyl-N',N"-diethyl-L-glutamic acid-1,5-diamide, N-acetyl-N',N',N",N"-tetramethyl-L-glutamic acid-1,5-diamide, N-acetyl-N',N',N",N"-tetraethyl-L-glutamic acid-1,5-diamide, N-acetyl-L-glutamic acid bis-1,5-morpholine amide, and N-acetyl-L-glutamic acid bis-1,5-piperidine amide, significantly lowered the ΔTEWL value compared with the control aqueous solution. Thus, the effects of inhibiting/ameliorating rough skin was confirmed.
On the other hand, N-acetyl glutamine as a comparative example did not show such effects, and the effects in N-acetyl glutamic acid was inferior to that of the compounds of the present invention.

### Example 3 Sensory irritation test

1 mL of the control aqueous solution and the sample aqueous solution prepared in Test Example 1 was applied on the left and right cheek, respectively, of one female sensory person (who has sensitive skin) using a cotton swab. Then, the irritating sensation was evaluated every 30 seconds for ten minutes immediately after the application, and a final evaluation was reported (n=2). The evaluation of the irritating sensation was based on the following rating criteria in four levels, and each average rating was calculated to be classified by the following criteria.

(Rating criteria)
3: The test cannot be continued because of extremely strong irritation such as tingly, burning, prickly, and itching sensation.
2: The use is intolerable because of strong irritation such as tingly, burning, prickly, and itching sensation.
1: The use is tolerable though slight irritation such as tingly, burning, prickly, and itching sensation is felt.
0: The irritation is not particularly felt.

(Evaluation criteria)
A: Average rating is less than 0.2
B: Average rating is 0.2 or greater to less than 1.0
C: Average rating is 1.0 or greater to less than 2.0
D: Average rating is 2.0 or greater

**Table 4**

| Sample | Concentration (mass%) | Evaluation |
|---|---|---|
| Control aqueous solution | - | A |
| N-carbamoyl-L-glutamic acid | 3 | A |
| N-benzyloxycarbonyl-L-glutamic acid | 3 | A |
| N-methyl-L-glutamic acid | 3 | A |
| N-methyl-DL-glutamic acid | 3 | A |
| N-acetyl-N'-methyl-L-glutamic acid-1-amide | 3 | A |
| N-acetyl-N'-ethyl-L-glutamic acid-1-amide | 3 | A |
| N-acetyl-N'-n-propyl-L-glutamic acid-1-amide | 3 | A |
| N-acetyl-N'-benzyl-L-glutamic acid-1-amide | 3 | A |
| N-acetyl-N'-cyclohexyl-L-glutamic acid-1-amide | 3 | A |
| N-acetyl-N'-cyclopentyl-L-glutamic acid-1-amide | 3 | A |
| N-acetyl-L-glutamic acid 1-pyrrolidine amide | 3 | A |
| N-acetyl-L-glutamic acid 1-piperidine amide | 3 | A |
| N-acetyl-L-glutamic acid 1-morpholine amide | 3 | A |
| N-benzoyl-N'-methyl-L-glutamic acid-1-amide | 3 | A |
| N-benzoyl-N'-ethyl-L-glutamic acid-1-amide | 3 | A |
| N-benzoyl-N'-n-propyl-L-glutamic acid-1-amide | 3 | A |
| N-benzoyl-L-glutamic acid 1-morpholine amide | 3 | A |
| N-acetyl-N',N"-dimethyl-L-glutamic acid-1,5-diamide | 3 | A |
| N-acetyl-N',N"-diethyl-L-glutamic acid-1,5-diamide | 3 | A |
| N-acetyl-N',N',N",N"-tetramethyl-L-glutamic acid-1,5-diamide | 3 | A |
| N-acetyl-N',N',N",N"-tetraethyl-L-glutamic acid-1,5-diamide | 3 | A |
| N-acetyl-L-glutamic acid bis-1,5-morpholine amide | 3 | A |
| N-acetyl-L-glutamic acid bis-1,5-piperidine amide | 3 | A |
| Γ-aminobutyric acid (comparative example) | 3 | C |

As is clear from Table 4, the glutamic acid derivatives according to the present invention, such as N-carbamoyl-L-glutamic acid, N-benzyloxycarbonyl-L-glutamic acid, N-methyl-L-glutamic acid, N-methyl-DL-glutamic acid, N-acetyl-N'-methyl-L-glutamic acid-1-amide, N-acetyl-N'-ethyl-L-glutamic acid-1-amide, N-acetyl-N'-n-propyl-L-gtutamic acid-1-amide, N-acetyl-N'-benzyl-L-glutamic acid-1-amide, N-acetyl-N'-cyclohexyl-L-glutamic acid-1-amide, N-acetyl-N'-cyclopentyl-L-glutamic acid-1-amide, N-acetyl-L-glutamic acid 1-pyrrolidine amide, N-acetyl-L-glutamic acid 1-piperidine amide, N-acetyl-L-glutamic acid 1-morpholine amide, N-benzoyl-N'-methyl-L-glutamic acid-1-amide, N-benzoyl-N'-ethyl-L-glutamic acid-1-amide, N-benzoyl-N'-n-propyl-L-glutamic acid-1-amide, N-benzoyl-L-glutamic acid 1-morpholine amide, N-acetyl-N',N"-dimethyl-L-glutamic acid-1,5-diamide, N-acetyl-N',N"-diethyl-L-glutamic acid-1,5-diamide, N-acetyl-N',N',N",N"-tetramethyl-L-glutamic acid-1,5-diamide, N-acetyl-N',N',N",N"-tetraethyl-L-glutamic acid-1,5-diamide, N-acetyl-L-glutamic acid bis-1,5-morpholine amide, and N-acetyl-L-glutamic acid bis-1,5-piperidine amide, had no problem in sensory irritation, and thus were confirmed to be high in safety.
On the other hand, γ-aminobutyric acid, which was disclosed as a conventional active substance, used as a comparative example resulted in giving sensory irritation to the test subject who had sensitive skin.

**Table 5**

| Sample | Concentration (mass%) | Evaluation |
|---|---|---|
| Control aqueous solution | - | A |
| N-amidino-L-glutamic acid | 3.0 | A |
| N-amidino-D-glutamic acid | 3.0 | A |
| N-amidino-DL-glutamic acid | 3.0 | A |
| Sodium N-amidino-L-glutamate | 3.0 | A |
| N-amidino-L-glutamic acid hydrochloride | 3.0 | A |
| γ-aminobutyric acid (comparative example) | 3.0 | C |

As is clear from Table 5, the N-amidinoglutamic acids according to the present invention, such as N-amidino-L-glutamic acid, N-amidino-D-glutamic acid, N-amidino-DL-glutamic acid, sodium N-amidino-L-glutamate, and N-amidino-L-glutamic acid hydrochloride, had no problem in sensory irritation, and thus were confirmed to be high in safety.
On the other hand, though γ-aminobutyric acid, which was disclosed as a conventional active substance, used as a comparative example resulted in giving sensory irritation to the test subject who had sensitive skin.

### Test Example 4 Inhibiting effect of increased production of inflammatory cytokine caused by oleic acid

Human normal keratinocytes (Strain No. 070413-902), bought from KURABO INDUSTRIES LTD., were incubated using KGM culture medium in a 75 cm² flask for 3 days. The cells were trypsinized according to a normal method, collected, and then inoculated into a 24 well plate (Coming Inc.) so as to be 40,000 cells per 0.5 ml of KGM culture medium. After incubation for another 3 days, oleic acid, or oleic acid and test compound, were added thereto. The concentrations of oleic acid and test compound were 2 x 10⁻³ % and 0.05 %, respectively. As a control, ethanol was added instead of oleic acid. After 8 hours, each culture was collected, and the amount of IL-1α was measured with a quantification kit (from R&D systems).

As a representative example, the result when N-carbamoyl-L-glutamic acid was used as the test compound is shown in Fig. 1.
As is clear from Fig. 1, it was found that the compound of the present invention inhibits the increased production of IL-1α, a kind of inflammatory cytokines, caused by oleic acid.

The following Table 6 shows the results when oleic acid (10 mM ethanol solution) and N-acetyl-N',N"-diethyl-L-glutamic acid-1,5-diamide (50 mM solution) were added so that the concentrations of oleic acid and N-acetyl-N',N"-diethyl-L-glutamic acid-1,5-diamide were 10 µM (2.8 x 10⁻⁴ %) and 500 µM (0.012 %), respectively. Also from Fig. 6, it can be understood that the compound of the present invention significantly inhibits the increased production of IL-1α, a kind of inflammatory cytokines, caused by oleic acid.

**Table 6**

| Sample | IL-1α (pmol/ml, Mean ± S.D.) |
|---|---|
| Solvent (ethanol) | 4.7 ± 1.5 |
| 10 µM oleic acid | 239.8 ± 1.3 |
| 10 µM oleic acid + 500 µM N-acetyl-N',N"-diethyl-L-glutamic acid-1,5-diamide | 225.5 ± 3.2** |

| | |
|---|---|
| **p < 0.01 (vs. oleic acid) | |

Hereinafter, external compositions for skin will be explained as preparation examples of the compositions according to the present invention. All of the external compositions for skin were prepared according to normal methods. The amounts are shown in mass%.

| Preparation Example 1 Lotion | |
|---|---|
| (1) 1,3-Butylene glycol | 6.0 |
| (2) Glycerin | 4.0 |
| (3) Oleyl alcohol | 0.1 |
| (4) POE(20)sorbitan monolaurate | 0.5 |
| (5) POE(15)lauryl alcohol ester | 0.5 |
| (6) Ethanol | 10.0 |
| (7) N-carbamoyl-L-glutamic acid | 1.0 |
| (8) Purified water | Balance |

| Preparation Example 2 Lotion | |
|---|---|
| (Alcohol phase) | |
| (1) Ethanol | 10.0 |
| (2) Oleyl alcohol | 0.1 |
| (3) POE(20)sorbitan monolaurate | 0.5 |
| (4) POE(15)lauryl ether | 0.5 |
| (5) Preservative | Q.S. |
| (6) Perfume | Q.S. |
| (7) N-benzyloxycarbonyl-L-glutamic acid | 0.7 |

| (Aqueous phase) | |
|---|---|
| (8) 1,3-Butylene glycol | 6.0 |
| (9) Glycerin | 4.0 |
| (10) Ion-exchanged water | Balance |

| Preparation Example 3 Lotion | |
|---|---|
| Ethyl alcohol | 5.0 |
| Glycerin | 1.0 |
| 1,3-Butylene glycol | 5.0 |
| Polyoxyethylene polyoxypropylene decyltetradecyl ether | 0.2 |
| Sodium hexametaphosphate | 0.03 |
| Trimethylglycine | 1.0 |
| Sodium polyaspartate | 0.1 |
| Dipotassium L-ascorbyl α-tocopheryl phosphate | 0.1 |
| Thiotaurine | 0.1 |
| N-acetyl-L-glutamic acid bis-1,5-piperidine amide | 1.0 |
| Glycyl glycine | 0.5 |
| Green tea extract | 0.1 |
| Peppermint extract | 0.1 |
| Iris florentina root extract | 0.1 |
| Trisodium EDTA | 0.1 |
| Carboxyvinyl polymer | 0.05 |
| Potassium hydroxide | 0.02 |
| Phenoxyethanol | Q.S. |
| Purified water | Balance |
| Perfume | Q.S. |

| Preparation Example 4 Lotion | |
|---|---|
| Glycerin | 2.0 |
| 1,3-Butylene glycol | 4.0 |
| Erythritol | 1.0 |
| N-methyl-L-glutamic acid | 1.0 |
| Polyoxyethylene methyl glucoside | 1.0 |
| Polyoxyethylene hydrogenated castor oil | 0.5 |
| Citric acid | 0.02 |
| Sodium citrate | 0.08 |
| Phenoxyethanol | Q.S. |
| Ethyl N-cocoyl-L-arginine DL-pyrrolidonecarboxylic acid | 0.1 |
| Purified water | Balance |

| Preparation Example 5 Lotion | |
|---|---|
| Glycerin | 1.0 |
| Dipropylene glycol | 12.0 |
| Ethanol | 8.0 |
| N-acetyl-N',N',N",N"-tetraethyl-L-glutamic acid-1,5-diamide | 2.0 |
| POE methyl glucoside | 3.0 |
| POE(24)POP(13)decyltetradecyl ether | 0.5 |
| Citric acid | 0.02 |
| Sodium citrate | 0.08 |
| Hydroxypropyl-β-cyclodextrin | 0.5 |
| Thiotaurine | 0.1 |
| Disodium adenosine triphosphate | 0.1 |
| Sodium hyaluronate | 0.01 |
| Trisodium EDTA | 0.01 |
| Hydroxyethyl cellulose | 0.1 |
| Paraben | Q.S. |
| Purified water | Balance |
| Perfume | Q.S. |

| Preparation Example 6 Cream | |
|---|---|
| (1) Stearic acid | 5.0 |
| (2) Stearyl alcohol | 4.0 |
| (3) Isopropyl myristate | 18.0 |
| (4) Glyceryl Monostearate | 3.0 |
| (5) Propylene glycol | 10.0 |
| (6) N-acetyl-L-glutamic acid bis-1,5-morpholine amide | 1.0 |
| (7) Potassium hydroxide | 0.2 |
| (8) Sodium bisulfite | 0.01 |
| (9) Preservative | Q.S. |
| (10) Perfume | Q.S. |
| (11) Ion-exchanged water | Balance |

| Preparation Example 7 Cream | |
|---|---|
| Stearic acid | 6.0 |
| Sorbitan monostearate | 2.0 |
| POE(20)sorbitan monostearate | 1.5 |
| Propylene glycol | 10.0 |
| Glycerin trioctanoate | 10.0 |
| Squalane | 5.0 |
| N-acetyl-N'-methyl-L-glutamic acid-1-amide | 0.1 |
| Glycyl glycine | 1.0 |
| Dihydrolipoic acid | 0.1 |
| Sodium bisulfite | 0.01 |
| Ethyl paraben | 0.3 |
| Perfume | Q.S. |
| Ion-exchanged water | Balance |

| Preparation Example 8 Cream | |
|---|---|
| Liquid paraffin | 10.0 |
| Dimethylpolysiloxane | 2.0 |
| Glycerin | 10.0 |
| 1,3-Butylene glycol | 2.0 |
| Erythritol | 1.0 |
| N-acetyl-N'-ethyl-L-glutamic acid-1-amide | 3.0 |
| Polyethylene glycol 1500 | 5.0 |
| Squalane | 15.0 |
| Pentaerythritol tetra-2-ethylhexanoate | 5.0 |
| Potassium hydroxide | 0.1 |
| Sodium hexametaphosphate | 0.05 |
| Tocopheryl acetate | 0.05 |
| Paraoxybenzoate | Q.S. |
| Hydroxypropyl methylcellulose | 0.3 |
| Polyvinyl alcohol | 0.1 |
| Carboxyvinyl polymer | 0.2 |
| Acrylic acid-alkyl methacrylate copolymer (Pemulen TR-2) | 0.1 |
| Purified water | Balance |

| Preparation Example 9 Cream | |
|---|---|
| Liquid paraffin | 8.0 |
| Petrolatum | 3.0 |
| Dimethylpolysiloxane | 2.0 |
| Stearyl alcohol | 3.0 |
| Behenyl alcohol | 2.0 |
| Glycerin | 5.0 |
| Dipropylene glycol | 4.0 |
| Trehalose | 1.0 |
| Pentaerythritol tetra-2-ethylhexanoate | 4.0 |
| Polyoxyethylene glyceryl monoisostearate | 2.0 |
| Polyoxyethylene glyceryl monostearate | 1.0 |
| Lipophilic glyceryl monostearate | 2.0 |
| Citric acid | 0.05 |
| Sodium citrate | 0.05 |
| Potassium hydroxide | 0.015 |
| Oil-soluble glycyrrhiza glabra extract | 0.1 |
| Retinol palmitate (megaunit) | 0.25 |
| Tocopheryl acetate | 0.1 |
| Paraoxybenzoate | Q.S. |
| Phenoxyethanol | Q.S. |
| Dibutylhydroxytoluene | Q.S. |
| Trisodium edetate | 0.05 |
| 4-t-Butyl-4'-methoxydibenzoylmethane | 0.01 |
| 2-Ethylhexyl p-methoxycinnamate | 0.1 |
| N-benzoyl-N'-ethyl-L-glutamic acid-1-amide | 1.0 |
| β-carotene | 0.01 |
| Polyvinyl alcohol | 0.5 |
| Hydroxyethyl cellulose | 0.5 |
| Carboxyvinyl polymer | 0.05 |
| Purified water | Balance |
| Perfume | Q.S. |

| Preparation Example 10 Cream | |
|---|---|
| A. Water phase | |
| Ion-exchanged water | Balance |
| Polyethylene glycol 400 | 0.1 |
| Glycerin | 12.0 |
| 1,3-Butylene glycol | 5.0 |
| Sodium edetate | 0.01 |
| Citric acid | 0.01 |
| Sodium citrate | 0.04 |
| Methylparaben | 0.1 |
| N-acetyl-N',N"-dimethyl-L-glutamic acid-1,5-diamide | 2.0 |
| Sodium N-stearoyl glutamate | 0.8 |

| B. Oil phase | |
|---|---|
| Liquid paraffin | 35.0 |
| Meadowfoam oil | 3.0 |
| Octyl methoxycinnamate | 3.0 |
| 2-Heptylundecanoic acid | 0.2 |
| Isostearic acid | 0.2 |
| Stearyl glycyrrhetinate | 0.02 |
| Perfume | 0.05 |

| Preparation Example 11 Cream | |
|---|---|
| α-Olefine oligomer | 10.0 |
| Petrolatum | 1.0 |
| Microcrystalline wax | 3.0 |
| Decamethylcyclopentasiloxane | 5.0 |
| Glycerin | 10.0 |
| Dipropylene glycol | 2.0 |
| 1,3-Butylene glycol | 2.0 |
| Erythritol | 2.0 |
| N-acetyl-N',N"-diethyl-L-glutamic acid-1,5-diamide | 3.0 |
| Squalane | 1.0 |
| Glycerin fatty acid eicosanedioic acid ester condensate | 0.1 |
| Isostearic acid | 1.0 |
| Cetyl 2-ethylhexanoate | 5.0 |
| Sodium chloride | 0.5 |
| Sodium hexametaphosphate | 0.05 |
| Stearyl glycyrrhetinate | 0.05 |
| Yeast extract | 0.1 |
| 4-(1-Pyrrolidine)butyric acid | 1.0 |
| Magnesium L-ascorbylphosphate | 2.0 |
| Tocopherol acetate | 0.5 |
| Thiotaurine | 0.1 |
| Sodium DL-pyrrolidonecarboxylate | 1.0 |
| Turmeric extract | 0.1 |
| Trisodium edetate | 0.1 |
| Dimethyldistearylammonium hectolite | 2.0 |
| Sodium carboxymethylcellulose | 0.1 |
| Camellia reticulata seed oil | 1.0 |
| Paraben | Q.S. |
| Purified water | Balance |
| Perfume | Q.S. |

| Preparation Example 12 Essence | |
|---|---|
| (Phase A) | |
| (1) Ethyl alcohol (95 %) | 10.0 |
| (2) POE(20)octyldodecanol | 1.0 |
| (3) Pantothenyl ethyl ether | 0.1 |
| (4) Glycyl glycine | 1.5 |
| (5) Methylparaben | 0.15 |

| (Phase B) | |
|---|---|
| (6) Potassium hydroxide | 0.1 |

| (Phase C) | |
|---|---|
| (7) Glycerin | 5.0 |
| (8) Dipropylene glycol | 10.0 |
| (9) N-acetyl-L-glutamic acid 1-morpholine amide | 0.3 |
| (10) Carboxyvinyl polymer | 0.2 |
| (11) Purified water | Balance |

| Preparation Example 13 Essence | |
|---|---|
| (Phase A) | |
| 95% Ethanol | 10.0 |
| POE(20)octyldodecanol | 1.0 |
| Methylparaben | 0.15 |
| Pantothenyl ethyl ether | 0.1 |
| N-acetyl-N'-benzyl-L-glutamic acid-1-amide | 2.5 |

| (Phase B) | |
|---|---|
| Potassium hydroxide | 0.1 |

| (Phase C) | |
|---|---|
| Glycerin | 5.0 |
| Dipropylene glycol | 10.0 |
| Sodium bisulfite | 0.03 |
| Carboxyvinyl polymer | 0.2 |
| Ion-exchanged water | Balance |

| Preparation Example 14 Milky lotion | |
|---|---|
| Decamethylcyclopentasiloxane | 15.0 |
| Trimethylsiloxysilicate | 5.0 |
| Polyoxyethylene-methylpolysiloxane copolymer | 5.0 |
| Glycerin | 5.0 |
| 1,3-Butylene glycol | 5.0 |
| Maltitol solution | 2.0 |
| Macadamia nuts oil | 2.0 |
| Squalane | 2.0 |
| Cholesteryl hydroxystearate | 0.5 |
| Cetyl 2-ethylhexanoate | 2.0 |
| N-acetyl-L-glutamic acid 1-piperidine amide | 2.0 |
| Distearyldimethylammonium chloride | 0.2 |
| Disodium L-ascorbicsulfate | 0.1 |
| Dipotassium L-ascorbyl α-tocopheryl phosphate | 0.1 |
| Tocopherol acetate | 0.05 |
| Fish collagen | 0.4 |
| Sodium chondroitin sulphate | 0.001 |
| Sodium hyaluronate | 0.1 |
| Trisodium edetate | 0.005 |
| Glyceryl di(p-methoxycinnamate) mono(2-ethylhexanoate) | 0.05 |
| Aluminum magnesium silicate | 0.3 |
| Paraben | Q.S. |
| Purified water | Balance |

| Preparation Example 15 Milky lotion | |
|---|---|
| Petrolatum | 5.0 |
| Dimethylpolysiloxane | 2.0 |
| Behenyl alcohol | 0.6 |
| Batyl alcohol | 0.5 |
| Dipropylene glycol | 2.0 |
| 1,3-Butylene glycol | 4.0 |
| Xylitol | 1.0 |
| N-acetyl-L-glutamic acid 1-pyrrolidine amide | 2.0 |
| Polyethylene glycol 1500 | 1.0 |
| Squalane | 5.0 |
| Glyceryl tri(2-ethylhexanoate) | 2.0 |
| Polyoxyethylene hydrogenated castor oil | 0.5 |
| Dipotassium glycyrrhizinate | 0.1 |
| Yeast extract | 0.1 |
| Paeonia albiflora root extract | 0.1 |
| Trisodium EDTA | 0.05 |
| Xanthan gum | 0.1 |
| Carboxyvinyl polymer | 0.15 |
| Purified water | Balance |
| Perfume | Q.S. |

| Preparation Example 16 Milky lotion | |
|---|---|
| Ion-exchanged water | Balance |
| Dynamite glycerin | 7.0 |
| Dipropylene glycol | 5.0 |
| 1,3-Butylene glycol | 2.0 |
| Acetylated hyaluronic acid | 0.002 |
| Carboxyvinyl polymer | 0.3 |
| Xanthan gum | 0.1 |
| Potassium hydroxide | 0.1 |
| Sodium N-stearoyl-L-glutamate | 0.1 |
| Pentaerythritol tetra-2-ethylhexanoate | 2.0 |
| N-acetyl-N'-ethyl-L-glutamic acid-1-amide | 1.0 |
| Squalane | 4.5 |
| Dextrin palmitate | 2.0 |
| Hydrogenated polyisobutene | 1.0 |
| Trisodium EDTA-2H₂O | 0.05 |
| Methylparaben | 0.15 |
| Ethylparaben | 0.1 |
| Antioxidant | Q.S. |
| Anti-foaming agent | Q.S. |

| Preparation Example 17 Gel | |
|---|---|
| (1) 95% Ethanol | 10.0 |
| (2) Dipropylene glycol | 15.0 |
| (3) POE(15)oleyl ether | 2.0 |
| (4) Sodium bisulfite | 0.03 |
| (5) N-acetyl-N'-cyclopentyl-L-glutamic acid-1-amide | 1.2 |
| (6) Carboxyvinyl polymer (Carbopol 941) | 1.0 |
| (7) Potassium hydroxide | 0.15 |
| (8) L-arginine | 0.1 |
| (9) Perfume | Q.S. |
| (10) Preservative | Q.S. |
| (11) Purified water | Balance |

| Preparation Example 18 Gel | |
|---|---|
| Glycerin | 2.0 |
| 1,3-Butylene glycol | 5.0 |
| Potassium hydroxide | 0.1 |
| Fish collagen | 20.0 |
| Glycyl glycine | 1.0 |
| N-acetyl-N',N'-dimethyl-L-glutamic acid-1-amide | 1.0 |
| N-benzenesulfonyl-γ-aminobutyric acid | 0.5 |
| Trisodium edetate | 0.05 |
| Carboxyvinyl polymer | 0.25 |
| Paraoxybenzoate | Q.S. |
| Purified water | Balance |

| Preparation Example 19 Gel | |
|---|---|
| Dimethylpolysiloxane | 5.0 |
| Glycerin | 2.0 |
| 1,3-Butylene glycol | 5.0 |
| Polyethylene glycol 1500 | 3.0 |
| Polyethylene glycol 20000 | 3.0 |
| Cetyl alcohol | 3.0 |
| Citric acid | 0.01 |
| Sodium citrate | 0.1 |
| Sodium hexametaphosphate | 0.1 |
| Dipotassium glycyrrhizinate | 0.1 |
| Ascorbic acid glucoside | 2.0 |
| N-benzoyl-N'-propyl-L-glutamic acid-1-amide | 1.0 |
| Tocopheryl acetate | 0.1 |
| Scutellaria baicalensis root extract | 0.1 |
| Saxifraga sarmentosa extract | 0.1 |
| Trisodium edetate | 0.1 |
| Xanthan gum | 0.3 |
| Acrylic acid-alkyl methacrylate copolymer (Pemulen TR-2) | 0.05 |
| Agar powder | 15.0 |
| Phenoxyethanol | Q.S. |
| Dibutylhydroxytoluene | Q.S. |
| Purified water | Balance |

| Preparation Example 20 Pack | |
|---|---|
| Ethanol | 3.0 |
| Glycerin | 5.0 |
| 1,3-Butylene glycol | 6.0 |
| Polyethylene glycol 1500 | 5.0 |
| Polyoxyethylene methyl glucoside | 2.0 |
| Glyceryl tri(2-ethylhexanoate) | 1.0 |
| Sodium hexametaphosphate | 0.05 |
| Hydroxypropyl-β-cyclodextrin | 0.1 |
| Dipotassium glycyrrhizinate | 0.1 |
| Eriobotrya japonica leaf extract | 0.1 |
| N-acetyl-N'-cyclopentyl-L-glutamic acid-1-amide | 1.8 |
| Sodium L-glutamate | 0.1 |
| Fennel extract | 0.1 |
| Hamamelis virginiana extract | 0.1 |
| Phellodendron extract | 0.1 |
| Rehmannia chinensis root extract | 0.1 |
| Eucalyptus globulus leaf oil | 0.05 |
| Dimorpholinopyridazinone | 0.1 |
| Xanthan gum | 0.05 |
| Carboxyvinyl polymer | 0.5 |
| Acrylic acid-alkyl methacrylate copolymer (Pemulen TR-1) | 0.05 |
| Potassium hydroxide | 0.05 |
| Phenoxyethanol | Q.S. |
| Purified water | Balance |

| Preparation Example 21 Peel-off type pack | |
|---|---|
| (Alcohol phase) | |
| 95% Ethanol | 10.0 |
| POE(15)oleyl ether | 2.0 |
| Preservative | Q.S. |
| Perfume | Q.S. |
| N-acetyl-N'-cyclopentyl-L-glutamic acid-1-amide | 1.8 |

| (Water phase) | |
|---|---|
| Glutathione | 3.0 |
| Arbutin | 3.0 |
| Polyvinyl alcohol | 12.0 |
| Polyethylene glycol 1500 | 1.0 |
| Ion-exchenged water | Balance |

| Preparation Example 22 Peel-off type pack | |
|---|---|
| Ethanol | 10.0 |
| 1,3-Butylene glycol | 6.0 |
| Polyethylene glycol 4000 | 2.0 |
| Olive oil | 1.0 |
| Macadamia nuts oil | 1.0 |
| N-acetyl-L-glutamic acid 1-pyrrolidine amide | 2.0 |
| Phytosteryl hydroxystearate | 0.05 |
| Lactic acid | 0.05 |
| Sodium lactate | 0.1 |
| Disodium L-ascorbicsulfate | 0.1 |
| Dipotassium L-ascorbyl α-tocopheryl phosphate | 0.1 |
| Vitamin E acetate | 0.1 |
| Fish collagen | 0.1 |
| Sodium chondroitin sulphate | 0.1 |
| Sodium carboxymethylcellulose | 0.2 |
| Polyvinyl alcohol | 12.0 |
| Paraoxybenzoate | Q.S. |
| Purified water | Balance |
| Perfume | Q.S. |

| Preparation Example 23 Powder-containing pack | |
|---|---|
| (Alcohol phase) | |
| 95% Ethanol | 2.0 |
| Preservative | Q.S. |
| Perfume | Q.S. |
| Coloring material | Q.S. |
| N-methyl-DL-glutamic acid | 0.7 |

| (Water phase) | |
|---|---|
| Propylene glycol | 7.0 |
| Zinc oxide | 25.0 |
| Kaolin | 20.0 |
| Ion-exchanged water | Balance |

| Preparation Example 24 Clay pack | |
|---|---|
| Ethanol | 3.0 |
| Stearyl alcohol | 1.0 |
| Behenyl alcohol | 1.0 |
| Glycerin | 10.0 |
| Dipropylene glycol | 5.0 |
| 1,3-Butylene glycol | 6.0 |
| Fructose | 0.1 |
| Hardened oil | 2.0 |
| Isostearic acid | 0.06 |
| Glyceryl monostearate, self-emulsifying | 12.0 |
| Polyoxyethylene glyceryl monostearate | 0.8 |
| Lauryl dimethylaminoacetic acid betain | 0.1 |
| N-acetyl-N',N',N",N"-tetraethyl-L-glutamic acid-1,5-diamide | 1.2 |
| Titanium oxide | 15.0 |
| Zinc oxide | 2.0 |
| Kaolin | 7.0 |
| L-arginine L-aspartate | 0.1 |
| Vitamin E acetate | 0.1 |
| Sodium hyaluronate | 0.1 |
| Trisodium EDTA | 0.1 |
| Bentonite | 3.0 |
| Paraoxybenzoate | Q.S. |
| Purified water | Balance |
| Perfume | Q.S. |

| Preparation Example 25 Compact powdery foundation | |
|---|---|
| Dimethylpolysiloxane | 5.0 |
| Isostearic acid | 0.5 |
| Diisostearyl malate | 1.0 |
| Glyceryl tri(2-ethylhexanoate) | 3.0 |
| N-benzoyl-L-glutamic acid 1-morpholine amide | 3.0 |
| Sorbitan sesquiisostearate | 1.0 |
| Spherical PMMA-coated mica | 4.0 |
| Particulate zinc oxide | 1.0 |
| Particulate titanium oxide | 3.0 |
| Synthetic fluorphlogopite | 1.0 |
| Metal soap treated talc | Balance |
| Spherical silica | 3.0 |
| Red iron oxide-coated titanated mica | 1.0 |
| Anhydrous silicic acid-coated mica | 6.0 |
| DL-α-tocopherol acetate | 0.1 |
| D-δ-tocopherol | 0.1 |
| Ethylparaben | Q.S. |
| Methyl bis(trimethylsiloxy)silylisopentyl trimethoxycinnamate | 0.1 |
| 2-Ethylhexyl p-methoxycinnamate | 3.0 |
| Spherical polyalkylacrylate powder | 2.0 |
| Polyalkylacrylate powder containing liquid paraffin | 4.0 |
| Methylhydrogenpolysiloxane-coated talc | 20.0 |
| Methylhydrogenpolysiloxane-coated sericite | 15.0 |
| Methylhydrogenpolysiloxane-coated titanium oxide | 10.0 |
| Methylhydrogenpolysiloxane-coated pigment (coloring material) | 5.0 |

| Preparation Example 26 Water-in-oil emulsion foundation | |
|---|---|
| Dimethylpolysiloxane | 15.0 |
| Decamethylcyclopentasiloxane | 20.0 |
| Polyoxyethylene-methylpolysiloxane copolymer | 5.0 |
| High molecular weight amino-modified silicone | 0.1 |
| Glycerin | 5.0 |
| N-acetyl-N'-n-propyl-L-glutamic acid-1-amide | 5.0 |
| 1,3-Butylene glycol | 10.0 |
| Palmitic acid | 0.5 |
| Cholesteryl macadamiate | 0.1 |
| Distearyldimethylammonium chloride | 0.2 |
| Alkyl-modified silicone resin-coated yellow iron oxide | 2.0 |
| Alkyl-modified silicone resin-coated red iron oxide | 1.0 |
| Alkyl-modified silicone resin-coated black iron oxide | 0.3 |
| Alkyl-modified silicone resin-coated titanium oxide | 10.0 |
| Alkyl-modified silicone resin-coated talc oxide | 15.0 |
| Silicone-coated spindle-shape titanium oxide | 3.0 |
| Sodium L-glutamate | 0.5 |
| DL-α-tocopherol acetate | 0.1 |
| Paraoxybenzoate | Q.S. |
| Methyl bis(trimethylsiloxy)silylisopentyl trimethoxycinnamate | 0.1 |
| Dimethyldistearylammonium hectorite | 15.0 |
| Spherical nylon powder | 1.0 |
| Purified water | Balance |
| Perfume | Q.S. |

All of the external compositions for skin of Preparation Examples 1 to 26 had excellent effects such as effects of inhibiting parakeratosis, shrinking pores, and inhibiting/ameliorating rough skin.

| Preparation Example 27 Lotion | |
|---|---|
| (1) 1,3-Butylene glycol | 6.0 |
| (2) Glycerin | 4.0 |
| (3) Oleyl alcohol | 0.1 |
| (4) POE(20)sorbitan monolaurate | 0.5 |
| (5) POE(15)lauryl alcohol ester | 0.5 |
| (6) Ethanol | 10.0 |
| (7) N-amidino-L-glutamic acid | 1.0 |
| (8) Purified water | Balance |

| Preparation Example 28 Lotion | |
|---|---|
| (Alcohol phase) | |
| (1) Ethanol | 10.0 |
| (2) Oleyl alcohol | 0.1 |
| (3) POE(20)sorbitan monolaurate | 0.5 |
| (4) POE(15)lauryl ether | 0.5 |
| (5) Preservative | Q.S. |
| (6) Perfume | Q.S. |
| (7) N-amidino-DL-glutamic acid | 0.7 |

| (Water phase) | |
|---|---|
| (8) 1,3-Butylene glycol | 6.0 |
| (9) Glycerin | 4.0 |
| (10) Ion-exchanged water | Balance |

| Preparation Example 29 Lotion | |
|---|---|
| Ethyl alcohol | 5.0 |
| Glycerin | 1.0 |
| 1,3-Butylene glycol | 5.0 |
| Polyoxyethylene polyoxypropylene decyltetradecyl ether | 0.2 |
| Sodium hexametaphosphate | 0.03 |
| Trimethylglycine | 1.0 |
| Sodium polyaspartate | 0.1 |
| Dipotassium L-ascorbyl α-tocopheryl phosphate | 0.1 |
| Thiotaurine | 0.1 |
| N-amidino-DL-glutamic acid | 1.0 |
| Glycyl glycine | 0.5 |
| Green tea extract | 0.1 |
| Peppermint extract | 0.1 |
| Iris florentina root extract | 0.1 |
| Trisodium EDTA | 0.1 |
| Carboxyvinyl polymer | 0.05 |
| Potassium hydroxide | 0.02 |
| Phenoxyethanol | Q.S. |
| Purified water | Balance |
| Perfume | Q.S. |

| Preparation Example 30 Lotion | |
|---|---|
| Glycerin | 2.0 |
| 1,3-Butylene glycol | 4.0 |
| Erythritol | 1.0 |
| N-amidino-D-glutamic acid | 1.0 |
| Polyoxyethylene methyl glucoside | 1.0 |
| Polyoxyethylene hydrogenated castor oil | 0.5 |
| Citric acid | 0.02 |
| Sodium citrate | 0.08 |
| Phenoxyethanol | Q.S. |
| Ethyl N-cocoyl-L-arginine DL-pyrrolidonecarboxylic acid | 0.1 |
| Purified water | Balance |

| Preparation Example 31 Lotion | |
|---|---|
| Glycerin | 1.0 |
| Dipropylene glycol | 12.0 |
| Ethanol | 8.0 |
| N-amidino-L-glutamic acid hydrochloride | 2.0 |
| POE methyl glucoside | 3.0 |
| POE(24)POP(13)decyltetradecyl ether | 0.5 |
| Citric acid | 0.02 |
| Sodium citrate | 0.08 |
| Hydroxypropyl-β-cyclodextrin | 0.5 |
| Thiotaurine | 0.1 |
| Disodium adenosine triphosphate | 0.1 |
| Sodium hyaluronate | 0.01 |
| Trisodium EDTA | 0.01 |
| Hydroxyethyl cellulose | 0.1 |
| Paraben | Q.S. |
| Purified water | Balance |
| Perfume | Q.S. |

| Preparation Example 32 Cream | |
|---|---|
| (1) Stearic acid | 5.0 |
| (2) Stearyl alcohol | 4.0 |
| (3) Isopropyl myristate | 18.0 |
| (4) Glyceryl Monostearate | 3.0 |
| (5) Propylene glycol | 10.0 |
| (6) Sodium N-amidino-L-glutamate | 1.0 |
| (7) Potassium hydroxide | 0.2 |
| (8) Sodium bisulfite | 0.01 |
| (9) Preservative | Q.S. |
| (10) Perfume | Q.S. |
| (11) Ion-exchanged water | Balance |

| Preparation Example 33 Cream | |
|---|---|
| Stearic acid | 6.0 |
| Sorbitan monostearate | 2.0 |
| POE(20)sorbitan monostearate | 1.5 |
| Propylene glycol | 10.0 |
| Glycerin trioctanoate | 10.0 |
| Squalane | 5.0 |
| Dipotassium N-amidino-L-glutamate | 0.1 |
| Glycyl glycine | 1.0 |
| Dihydrolipoic acid | 0.1 |
| Sodium bisulfite | 0.01 |
| Ethyl paraben | 0.3 |
| Perfume | Q.S. |
| Ion-exchanged water | Balance |

| Preparation Example 34 Cream | |
|---|---|
| Liquid paraffin | 10.0 |
| Dimethylpolysiloxane | 2.0 |
| Glycerin | 10.0 |
| 1,3-Butylene glycol | 2.0 |
| Erythritol | 1.0 |
| Sodium N-amidino-DL-glutamate | 3.0 |
| Polyethylene glycol 1500 | 5.0 |
| Squalane | 15.0 |
| Pentaerythritol tetra-2-ethylhexanoate | 5.0 |
| Potassium hydroxide | 0.1 |
| Sodium hexametaphosphate | 0.05 |
| Tocopheryl acetate | 0.05 |
| Paraoxybenzoate | Q.S. |
| Hydroxypropyl methylcellulose | 0.3 |
| Polyvinyl alcohol | 0.1 |
| Carboxyvinyl polymer | 0.2 |
| Acrylic acid-alkyl methacrylate copolymer (PemulenTR-2) | 0.1 |
| Purified water | Balance |

| Preparation Example 35 Cream | |
|---|---|
| Liquid paraffin | 8.0 |
| Petrolatum | 3.0 |
| Dimethylpolysiloxane | 2.0 |
| Stearyl alcohol | 3.0 |
| Behenyl alcohol | 2.0 |
| Glycerin | 5.0 |
| Dipropylene glycol | 4.0 |
| Trehalose | 1.0 |
| Pentaerythritol tetra-2-ethylhexanoate | 4.0 |
| Polyoxyethylene glyceryl monoisostearate | 2.0 |
| Polyoxyethylene glyceryl monostearate | 1.0 |
| Lipophilic glyceryl monostearate | 2.0 |
| Citric acid | 0.05 |
| Sodium citrate | 0.05 |
| Potassium hydroxide | 0.015 |
| Oil-soluble glycyrrhiza glabra extract | 0.1 |
| Retinol palmitate (megaunit) | 0.25 |
| Tocopheryl acetate | 0.1 |
| Paraoxybenzoate | Q.S. |
| Phenoxyethanol | Q.S. |
| Dibutylhydroxytoluene | Q.S. |
| Trisodium edetate | 0.05 |
| 4-t-Butyl-4'-methoxydibenzoylmethane | 0.01 |
| 2-Ethylhexyl p-methoxycinnamate | 0.1 |
| N-amidino-L-glutamic acid maleate | 1.0 |
| β-carotene | 0.01 |
| Polyvinyl alcohol | 0.5 |
| Hydroxyethyl cellulose | 0.5 |
| Carboxyvinyl polymer | 0.05 |
| Purified water | Balance |
| Perfume | Q.S. |

| Preparation Example 36 Cream | |
|---|---|
| A. Water phase | |
| Ion-exchanged water | Balance |
| Polyethylene glycol 400 | 0.1 |
| Glycerin | 12.0 |
| Glycyl glycine | 1.0 |
| 1,3-Butylene glycol | 5.0 |
| Sodium edetate | 0.01 |
| Citric acid | 0.01 |
| Sodium citrate | 0.04 |
| Methylparaben | 0.1 |
| N-amidino-L-glutamic acid | 2.0 |
| Sodium N-stearoylglutamate | 0.8 |

| B. Oil phase | |
|---|---|
| Liquid paraffin | 35.0 |
| Meadowfoam oil | 3.0 |
| Octyl methoxycinnamate | 3.0 |
| 2-Heptylundecanoic acid | 0.2 |
| Isostearic acid | 0.2 |
| Stearyl glycyrrhetinate | 0.02 |
| Perfume | 0.05 |

| Preparation Example 37 Cream | |
|---|---|
| α-olefine oligomer | 10.0 |
| Petrolatum | 1.0 |
| Microcrystalline wax | 3.0 |
| Decamethylcyclopentasiloxane | 5.0 |
| Glycerin | 10.0 |
| Dipropylene glycol | 2.0 |
| 1,3-Butylene glycol | 2.0 |
| Glycyl glycine | 0.5 |
| Erythritol | 2.0 |
| N-amidino-L-glutamic acid | 3.0 |
| N-benzenesulfonyl-L-glutamic acid | 1.0 |
| Squalane | 1.0 |
| Glycerin fatty acid ester eicosanedioic acid condensate | 0.1 |
| Isostearic acid | 1.0 |
| Cetyl 2-ethylhexanoate | 5.0 |
| Sodium chloride | 0.5 |
| Sodium hexametaphosphate | 0.05 |
| Stearyl glycyrrhetinate | 0.05 |
| Yeast extract | 0.1 |
| 4-(1-Pyrrolidine)butyric acid | 1.0 |
| Magnesium L-ascorbylphosphate | 2.0 |
| Tocopherol acetate | 0.5 |
| Thiotaurine | 0.1 |
| Sodium DL-pyrrolidonecarboxylate | 1.0 |
| Turmeric extract | 0.1 |
| Trisodium edetate | 0.1 |
| Dimethyldistearylammonium hectolite | 2.0 |
| Sodium carboxymethylcellulose | 0.1 |
| Camellia reticulata seed oil | 1.0 |
| Paraben | Q.S. |
| Purified water | Balance |
| Perfume | Q.S. |

| Preparation Example 38 Essence | |
|---|---|
| (Phase A) | |
| (1) Ethyl alcohol (95%) | 10.0 |
| (2) POE(20)octyldodecanol | 1.0 |
| (3) Pantothenyl ethyl ether | 0.1 |
| (4) Glycyl glycine | 1.5 |
| (5) Methylparaben | 0.15 |

| (Phase B) | |
|---|---|
| (6) Potassium hydroxide | 0.1 |

| (Phase C) | |
|---|---|
| (7) Glycerin | 5.0 |
| (8) Dipropylene glycol | 10.0 |
| (9) Dipotassium N-amidino-DL-glutamate | 0.3 |
| (10) Carboxyvinyl polymer | 0.2 |
| (11) Purified water | Balance |

| Preparation Example 39 Essence | |
|---|---|
| (Phase A) | |
| 95% Ethanol | 10.0 |
| POE(20)octyldodecanol | 1.0 |
| Methylparaben | 0.15 |
| Pantothenyl ethyl ether | 0.1 |
| Sodium N-amidino-D-glutamate | 2.5 |

| (Phase B) | |
|---|---|
| Potassium hydroxide | 0.1 |

| (Phase C) | |
|---|---|
| Glycerin | 5.0 |
| Dipropylene glycol | 10.0 |
| Sodium bisulfite | 0.03 |
| Carboxyvinyl polymer | 0.2 |
| Ion-exchanged water | Balance |

| Preparation Example 40 Milky lotion | |
|---|---|
| Decamethylcyclopentasiloxane | 15.0 |
| Trimethylsiloxysilicate | 5.0 |
| Polyoxyethylene-methylpolysiloxane copolymer | 5.0 |
| Glycerin | 5.0 |
| 1,3-Butylene glycol | 5.0 |
| Maltitol solution | 2.0 |
| Macadamia nuts oil | 2.0 |
| Squalane | 2.0 |
| Cholesteryl hydroxystearate | 0.5 |
| Cetyl 2-ethylhexanoate | 2.0 |
| N-amidino-D-glutamic acid acetate | 2.0 |
| Distearyldimethylammonium chloride | 0.2 |
| Disodium L-ascorbicsulfate | 0.1 |
| Dipotassium L-ascorbyl α-tocopheryl phosphate | 0.1 |
| Tocopherol acetate | 0.05 |
| Fish collagen | 0.4 |
| Sodium chondroitin sulphate | 0.001 |
| Sodium hyaluronate | 0.1 |
| Trisodium edetate | 0.005 |
| Glyceryl di(p-methoxycinnamate) mono(2-ethylhexanoate) | 0.05 |
| Aluminum magnesium silicate | 0.3 |
| Paraben | Q.S. |
| Purified water | Balance |

| Preparation Example 41 Milky lotion | |
|---|---|
| Petrolatum | 5.0 |
| Dimethylpolysiloxane | 2.0 |
| Behenyl alcohol | 0.6 |
| Batyl alcohol | 0.5 |
| Dipropylene glycol | 2.0 |
| 1,3-Butylene glycol | 4.0 |
| Xylitol | 1.0 |
| Dipotassium N-amidino-L-glutamate | 2.0 |
| Disodium N-amidino-D-glutamate | 1.0 |
| Polyethylene glycol 1500 | 1.0 |
| Squalane | 5.0 |
| Glyceryl tri(2-ethylhexanoate) | 2.0 |
| Polyoxyethylene hydrogenated castor oil | 0.5 |
| Dipotassium glycyrrhizinate | 0.1 |
| Yeast extract | 0.1 |
| Paeonia albiflora root extract | 0.1 |
| Trisodium EDTA | 0.05 |
| Xanthan gum | 0.1 |
| Carboxyvinyl polymer | 0.15 |
| Purified water | Balance |
| Perfume | Q.S. |

| Preparation Example 42 Milky lotion | |
|---|---|
| Ion-exchanged water | Balance |
| Dynamite glycerin | 7.0 |
| Dipropylene glycol | 5.0 |
| 1,3-Butylene glycol | 2.0 |
| Acetylated hyaluronic acid | 0.002 |
| Carboxyvinyl polymer | 0.3 |
| Xanthan gum | 0.1 |
| Potassium hydroxide | 0.1 |
| Sodium N-stearoyl-L-glutamate | 0.1 |
| Pentaerythritol tetra-2-ethylhexanoate | 2.0 |
| N-amidino-L-glutamic acid | 1.0 |
| Squalane | 4.5 |
| Dextrin palmitate | 2.0 |
| Hydrogenated polyisobutene | 1.0 |
| Trisodium EDTA-2H₂O | 0.05 |
| Methylparaben | 0.15 |
| Ethylparaben | 0.1 |
| Antioxidant | Q.S. |
| Anti-foaming agent | Q.S. |

| Preparation Example 43 Gel | |
|---|---|
| (1) 95% Ethanol | 10.0 |
| (2) Dipropylene glycol | 15.0 |
| (3) POE(15)oleyl ether | 2.0 |
| (4) Sodium bisulfite | 0.03 |
| (5) Dipotassium N-amidino-L-glutamate succinate | 1.2 |
| (6) Carboxyvinyl polymer (Carbopol 941) | 1.0 |
| (7) Potassium hydroxide | 0.15 |
| (8) L-arginine | 0.1 |
| (9) Perfume | Q.S. |
| (10) Preservative | Q.S. |
| (11) Purified water | Balance |

| Preparation Example 44 Gel | |
|---|---|
| Glycerin | 2.0 |
| 1,3-Butylene glycol | 5.0 |
| Potassium hydroxide | 0.1 |
| Fish collagen | 20.0 |
| N-amidino-D-glutamic acid | 1.0 |
| N-benzenesulfonyl-γ-aminobutyric acid | 0.5 |
| Trisodium edetate | 0.05 |
| Carboxyvinyl polymer | 0.25 |
| Paraoxybenzoate | Q.S. |
| Purified water | Balance |

| Preparation Example 45 Gel | |
|---|---|
| Dimethylpolysiloxane | 5.0 |
| Glycerin | 2.0 |
| 1,3-Butylene glycol | 5.0 |
| Polyethylene glycol 1500 | 3.0 |
| Polyethylene glycol 20000 | 3.0 |
| Cetyl alcohol | 3.0 |
| Citric acid | 0.01 |
| Sodium citrate | 0.1 |
| Sodium hexametaphosphate | 0.1 |
| Dipotassium glycyrrhizinate | 0.1 |
| Ascorbic acid glucoside | 2.0 |
| N-amidino-L-glutamic acid | 1.0 |
| Tocopheryl acetate | 0.1 |
| Scutellaria baicalensis root extract | 0.1 |
| Saxifraga sarmentosa extract | 0.1 |
| Trisodium edetate | 0.1 |
| Xanthan gum | 0.3 |
| Acrylic acid-alkyl methacrylate copolymer (Pemulen TR-2) | 0.05 |
| Agar powder | 15.0 |
| Phenoxyethanol | Q.S. |
| Dibutylhydroxytoluene | Q.S. |
| Purified water | Balance |

| Preparation Example 46 Pack | |
|---|---|
| Ethanol | 3.0 |
| Glycerin | 5.0 |
| 1,3-Butylene glycol | 6.0 |
| Polyethylene glycol 1500 | 5.0 |
| Polyoxyethylene methyl glucoside | 2.0 |
| Glyceryl tri(2-ethylhexanoate) | 1.0 |
| Sodium hexametaphosphate | 0.05 |
| Hydroxypropyl-β-cyclodextrin | 0.1 |
| Dipotassium glycyrrhizinate | 0.1 |
| Eriobotrya japonica leaf extract | 0.1 |
| N-amidino-DL-glutamic acid EDTA salt | 1.8 |
| Sodium L-glutamate | 0.1 |
| Fennel extract | 0.1 |
| Hamamelis virginiana extract | 0.1 |
| Phellodendron extract | 0.1 |
| Rehmannia chinensis root extract | 0.1 |
| Eucalyptus globulus leaf oil | 0.05 |
| Dimorpholinopyridazinone | 0.1 |
| Xanthan gum | 0.05 |
| Carboxyvinyl polymer | 0.5 |
| Acrylic acid-alkyl methacrylate copolymer (Pemulen TR-1) | 0.05 |
| Potassium hydroxide | 0.05 |
| Phenoxyethanol | Q.S. |
| Purified water | Balance |

| Preparation Example 47 Peel-off type pack | |
|---|---|
| (Alcohol phase) | |
| 95% Ethanol | 10.0 |
| POE(15)oleyl ether | 2.0 |
| Preservative | Q.S. |
| Perfume | Q.S. |
| N-amidino-L-glutamic acid | 1.8 |

| (Water phase) | |
|---|---|
| Glutathione | 3.0 |
| Arbutin | 3.0 |
| Polyvinyl alcohol | 12.0 |
| Polyethylene glycol 1500 | 1.0 |
| Ion-exchanged water | Balance |

| Preparation Example 48 Peel-off type pack | |
|---|---|
| Ethanol | 10.0 |
| 1,3-Butylene glycol | 6.0 |
| Polyethylene glycol 4000 | 2.0 |
| Olive oil | 1.0 |
| Macadamia nuts oil | 1.0 |
| N-amidino-DL-glutamic acid | 2.0 |
| Phytosteryl hydroxystearate | 0.05 |
| Lactic acid | 0.05 |
| Sodium lactate | 0.1 |
| Disodium L-ascorbicsulfate | 0.1 |
| Dipotassium L-ascorbyl α-tocopheryl phosphate | 0.1 |
| Vitamin E acetate | 0.1 |
| Fish collagen | 0.1 |
| Sodium chondroitin sulphate | 0.1 |
| Sodium carboxymethylcellulose | 0.2 |
| Polyvinyl alcohol | 12.0 |
| Paraoxybenzoate | Q.S. |
| Purified water | Balance |
| Perfume | Q.S. |

| Preparation Example 49 Powder-containing pack | |
|---|---|
| (Alcohol phase) | |
| 95% Ethanol | 2.0 |
| Preservative | Q.S. |
| Perfume | Q.S. |
| Coloring material | Q.S. |
| N-amidino-D-glutamic acid oxalate | 0.7 |

| (Water phase) | |
|---|---|
| Propylene glycol | 7.0 |
| Zinc oxide | 25.0 |
| Kaolin | 20.0 |
| Ion-exchanged water | Balance |

| Preparation Example 50 Clay pack | |
|---|---|
| Ethanol | 3.0 |
| Stearyl alcohol | 1.0 |
| Behenyl alcohol | 1.0 |
| Glycerin | 10.0 |
| Dipropylene glycol | 5.0 |
| 1,3-Butylene glycol | 6.0 |
| Fructose | 0.1 |
| Hardened oil | 2.0 |
| Isostearic acid | 0.06 |
| Glyceryl monostearate, self-emulsifying | 12.0 |
| Polyoxyethylene glyceryl monostearate | 0.8 |
| Lauryl dimethylaminoacetic acid betain | 0.1 |
| N-amidino-L-glutamic acid | 1.2 |
| Titanium oxide | 15.0 |
| Zinc oxide | 2.0 |
| Kaolin | 7.0 |
| L-arginine L-aspartate | 0.1 |
| Vitamin E acetate | 0.1 |
| Sodium hyaluronate | 0.1 |
| Trisodium EDTA | 0.1 |
| Bentonite | 3.0 |
| Paraoxybenzoate | Q.S. |
| Purified water | Balance |
| Perfume | Q.S. |

| Preparation Example 51 Compact powdery foundation | |
|---|---|
| Dimethylpolysiloxane | 5.0 |
| Isostearic acid | 0.5 |
| Diisostearyl malate | 1.0 |
| Glyceryl tri(2-ethylhexanoate) | 3.0 |
| N-amidino-L-glutamic acid sulfate | 3.0 |
| Sorbitan sesquiisostearate | 1.0 |
| Spherical PMMA-coated mica | 4.0 |
| Particulate zinc oxide | 1.0 |
| Particulate titanium oxide | 3.0 |
| Synthetic fluorphlogopite | 1.0 |
| Metal soap treated talc | Balance |
| Spherical silica | 3.0 |
| Red iron oxide-coated titanated mica | 1.0 |
| Anhydrous silicic acid-coated mica | 6.0 |
| DL-α-tocopherol acetate | 0.1 |
| D-δ-tocopherol | 0.1 |
| Ethylparaben | Q.S. |
| Methyl bis(trimethylsiloxy)silylisopentyl trimethoxycinnamate | 0.1 |
| 2-Ethylhexyl p-methoxycinnamate | 3.0 |
| Spherical polyalkylacrylate powder | 2.0 |
| Polyalkylacrylate powder containing liquid paraffin | 4.0 |
| Methylhydrogenpolysiloxane-coated talc | 20.0 |
| Methylhydrogenpolysiloxane-coated sericite | 15.0 |
| Methylhydrogenpolysiloxane-coated titanium oxide | 10.0 |
| Methylhydrogenpolysiloxane-coated pigment (coloring material) | 5.0 |

| Preparation Example 52 Water-in-oil emulsion foundation | |
|---|---|
| Dimethylpolysiloxane | 15.0 |
| Decamethylcyclopentasiloxane | 20.0 |
| Polyoxyethylene-methylpolysiloxane copolymer | 5.0 |
| High molecular weight amino-modified silicone | 0.1 |
| Glycerin | 5.0 |
| N-amidino-L-glutamic acid triethanolamine salt | 5.0 |
| 1,3-Butylene glycol | 10.0 |
| Palmitic acid | 0.5 |
| Cholesteryl macadamiate | 0.1 |
| Distearyldimethylammonium chloride | 0.2 |
| Alkyl-modified silicone resin-coated yellow iron oxide | 2.0 |
| Alkyl-modified silicone resin-coated red iron oxide | 1.0 |
| Alkyl-modified silicone resin-coated black iron oxide | 0.3 |
| Alkyl-modified silicone resin-coated titanium oxide | 10.0 |
| Alkyl-modified silicone resin-coated talc oxide | 15.0 |
| Silicone-coated spindle-shape titanium oxide | 3.0 |
| Sodium L-glutamate | 0.5 |
| DL-α-tocopherol acetate | 0.1 |
| Paraoxybenzoate | Q.S. |
| Methyl bis(trimethylsiloxy)silylisopentyl trimethoxycinnamate | 0.1 |
| Dimethyldistearylammonium hectorite | 15.0 |
| Spherical nylon powder | 1.0 |
| Purified water | Balance |
| Perfume | Q.S. |

Preparation Examples 27 to 52 are Preparation Examples of the external composition for skin for inhibiting parakeratosis or shrinking pores, containing N-amidinoglutamic acid derivatives or the salts thereof. All of the external compositions for skin of Preparation Examples 27 to 52 had excellent effects of inhibiting parakeratosis and shrinking pores.

## Claims

1. A parakeratosis inhibitor or pore-shrinking agent comprising, as an active ingredient, one or more compounds selected from the group consisting of glutamic acid derivatives represented by formula (1) or (2) and the salts thereof: 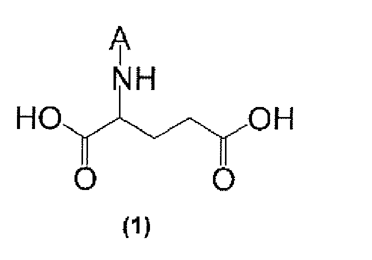 wherein A represents a carbamoyl group, a benzyloxycarbonyl group, an alkyl group having 1 to 3 carbon atoms, an allyl group, or an amidino group; 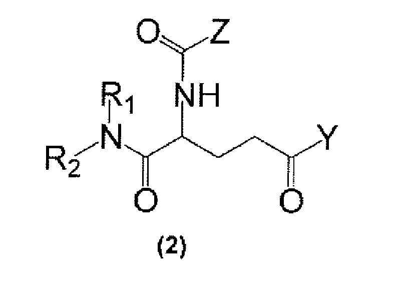
wherein Z represents an alkyl group having 1 to 4 carbon atoms, an alkenyl group having 2 to 4 carbon atoms, a benzyloxy group, or a group represented by formula (3) or (4);
Y represents a hydroxyl group, an alkyloxy group having 1 to 3 carbon atoms, an allyloxy group, or NR₃R₄; with the proviso that Y is not NR₃R₄ when Z is a group represented by the formula (4);
R₁ to R₄ each independently represents a hydrogen atom, an alkyl group having 1 to 3 carbon atoms, an allyl group, a cycloalkyl group having 3 to 7 carbon atoms, a cycloalkenyl group having 3 to 7 carbon atoms, a phenyl group, or a benzyl group, or R₁ and R₂, and/or R₃ and R₄, independently form a heterocycle having 6 carbon atoms or less in total together with the nitrogen atom to which they are bonded, wherein
the heterocycle may include an oxygen atom,
in R₁ to R₄, the alkyl group having 1 to 3 carbon atoms, the allyl group, the cycloalkyl group having 3 to 7 carbon atoms, the cycloalkenyl group having 3 to 7 carbon atoms, the phenyl group, the benzyl group, or the heterocycle may have, as a substituent, a hydroxyl group, an alkyloxy group having 1 to 3 carbon atoms, or an allyloxy group, and
in R₁ to R₄, the cycloalkyl group having 3 to 7 carbon atoms, the cycloalkenyl group having 3 to 7 carbon atoms, the phenyl group, the benzyl group, or the heterocycle may have, as a substituent, an alkyl group having 1 to 3 carbon atoms; 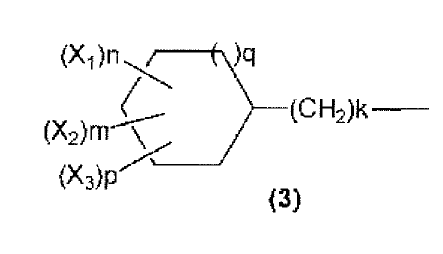
wherein X₁, X₂, and X₃ each independently represents an alkyl group having 1 to 4 carbon atoms, an alkenyl group having 1 to 4 carbon atoms, an alkyloxy group having 1 to 4 carbon atoms, an alkenyloxy group having 2 to 4 carbon atoms, a hydroxyl group, an amino group, an alkylamino group having 1 to 4 carbon atoms, a fluorine atom, or a trifluoromethyl group;
n, m, and p each independently represents an integer of 0 to 3;
k and q each independently represents an integer of 0 to 2; and 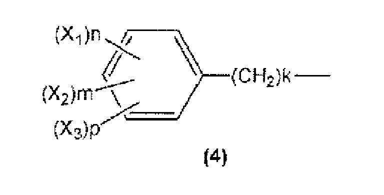
wherein X₁, X₂, X₃, k, n, m, and p are as defined in the formula (3).

2. The parakeratosis inhibitor or pore-shrinking agent of claim 1, comprising, as an active ingredient, one or more compounds selected from the group consisting of glutamic acid derivatives represented by formula (1a) and the salts thereof: 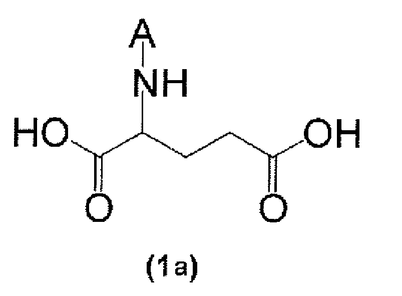 wherein A represents a carbamoyl group, a benzyloxycarbonyl group, an alkyl group having 1 to 3 carbon atoms, or an allyl group.

3. The parakeratosis inhibitor or pore-shrinking agent of claim 2, comprising, as an active ingredient, one or more compounds selected from the group consisting of N-carbamoylglutamic acid, N-benzyloxycarbonylglutamic acid, N-methylglutamic acid, and the salts thereof.

4. The parakeratosis inhibitor or pore-shrinking agent of claim 1, comprising, as an active ingredient, one or more compounds selected from the group consisting of glutamic acid derivatives represented by the formula (2) and the salts thereof.

5. The parakeratosis inhibitor or pore-shrinking agent of claim 4, wherein Z is a methyl group or a phenyl group.

6. The parakeratosis inhibitor or pore-shrinking agent of claim 4 or 5, wherein Y is a hydroxyl group or NR₃R₄.

7. The parakeratosis inhibitor or pore-shrinking agent of any of claims 4 to 6, wherein Y is the same as NR₁R₂.

8. The parakeratosis inhibitor or pore-shrinking agent of any of claims 4 to 7, wherein at least one of R₁ and R₂, and/or at least one of R₃ and R₄, each independently represents a hydrogen atom, an alkyl group having 1 to 3 carbon atoms, a cycloalkyl group having 3 to 7 carbon atoms, or a benzyl group.

9. The parakeratosis inhibitor or pore-shrinking agent of any of claims 4 to 8, wherein at least one of R1 and R₂, and/or at least one of R₃ and R₄, each independently represents a hydrogen atom, a methyl group, or an ethyl group.

10. The parakeratosis inhibitor or pore-shrinking agent of any of claims 4 to 7, wherein R₁ and R₂, and/or R₃ and R₄, independently form the heterocycle having 6 carbon atoms or less in total together with the nitrogen atom to which they are bonded.

11. The parakeratosis inhibitor or pore-shrinking agent of claim 4, comprising, as an active ingredient, one or more compounds selected from N-acetyl-N'-methylglutamic acid-1-amide, N-acetyl-N'-ethylglutamic acid-1-amide, N-acetyl-N'-n-propylglutamic acid-1-amide, N-acetyl-N'-benzylglutamic acid-1-amide, N-acetyl-N'-cyclohexylglutamic acid-1-amide, N-acetyl-N'-cyclopentylglutamic acid-1-amide, N-acetylglutamic acid 1-pyrrolidine amide, N-acetylglutamic acid 1-piperidine amide, N-acetylglutamic acid 1-morpholine amide, N-benzoyl-N'-methylglutamic acid-1-amide, N-benzoyl-N'-ethylglutamic acid-1-amide, N-benzoyl-N'-n-propylglutamic acid-1-amide, N-benzoylglutamic acid 1-morpholine amide, N-acetyl-N',N"-dimethylglutamic acid-1,5-diamide, N-acetyl-N',N"-diethylglutamic acid-1,5-diamide, N-acetyl-N',N',N",N"-tetramethylglutamic acid-1,5-diamide, N-acetyl-N',N',N",N"-tetraethylglutamic acid-1,5-diamide, N-acetylglutamic acid bis-1,5-morpholine amide, N-acetylglutamic acid bis-1,5-piperidine amide, and the salts thereof.

12. The parakeratosis inhibitor or pore-shrinking agent of claim 1, comprising, as an active ingredient, one or more compounds selected from the group consisting of N-amidinoglutamic acid represented by formula (1b) and the salt thereof.

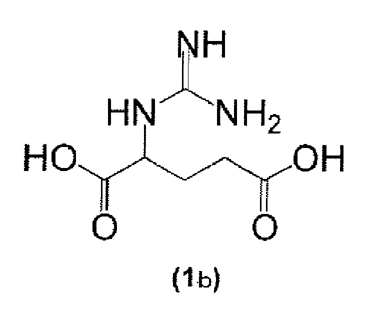

13. The parakeratosis inhibitor or pore-shrinking agent of claim 12, comprising, as an active ingredient, one or more compounds selected from the group consisting of N-amidino-L-glutamic acid (S-2-guanidinoglutaric acid) and the salt thereof.

14. An external composition for skin, comprising the parakeratosis inhibitor or pore-shrinking agent of any of claims 1 to 13.

15. A rough skin inhibiting or ameliorating agent, comprising, as an active ingredient, one or more compounds selected from the group consisting of glutamic acid derivatives represented by formula (1a) or (2) and the salts thereof: 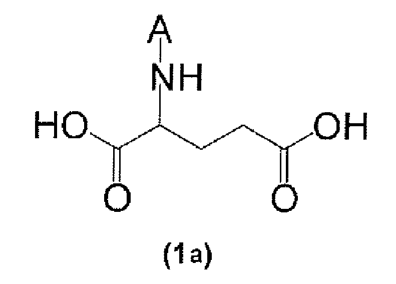 wherein A represents a carbamoyl group, a benzyloxycarbonyl group, an alkyl group having 1 to 3 carbon atoms, or an allyl group; 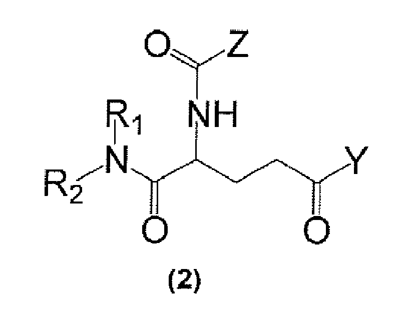
wherein Z represents an alkyl group having 1 to 4 carbon atoms, an alkenyl group having 2 to 4 carbon atoms, a benzyloxy group, or a group represented by formula (3) or (4);
Y represents a hydroxyl group, an alkyloxy group having 1 to 3 carbon atoms, an allyloxy group, or NR₃R₄, with the proviso that Y is not NR₃R₄ when Z is a group represented by the formula (4);
R₁ to R₄ each independently represents a hydrogen atom, an alkyl group having 1 to 3 carbon atoms, an allyl group, a cycloalkyl group having 3 to 7 carbon atoms, a cycloalkenyl group having 3 to 7 carbon atoms, a phenyl group, or a benzyl group, or R₁ and R₂, and/or R₃ and R₄, independently form a heterocycle having 6 carbon atoms or less in total together with the nitrogen atom to which they are bonded; wherein
the heterocycle may include an oxygen atom;
in R₁ to R₄, the alkyl group having 1 to 3 carbon atoms, the allyl group, the cycloalkyl group having 3 to 7 carbon atoms, the cycloalkenyl group having 3 to 7 carbon atoms, the phenyl group, the benzyl group, or the heterocycle may have, as a substituent, a hydroxyl group, an alkyloxy group having 1 to 3 carbon atoms, or an allyloxy group, and
in R₁ to R₄, the cycloalkyl group having 3 to 7 carbon atoms, the cycloalkenyl group having 3 to 7 carbon atoms, the phenyl group, the benzyl group, or the heterocycle may have, as a substituent, an alkyl group having 1 to 3 carbon atoms; 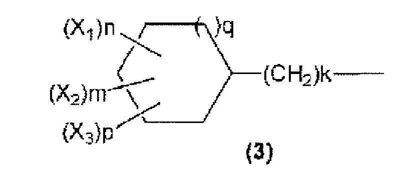
wherein X₁, X₂, and X₃ each independently represents an alkyl group having 1 to 4 carbon atoms, an alkenyl group having 1 to 4 carbon atoms, an alkyloxy group having 1 to 4 carbon atoms, an alkenyloxy group having 2 to 4 carbon atoms, a hydroxyl group, an amino group, an alkylamino group having 1 to 4 carbon atoms, a fluorine atom, or a trifluoromethyl group;
n, m, and p each independently represents an integer of 0 to 3;
k and q each independently represents an integer of 0 to 2; and 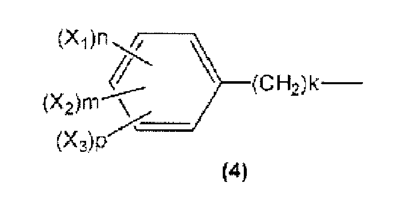
wherein X₁, X₂, X₃, k, n, m, and p are as defined in the formula (3).

16. The rough skin inhibiting or ameliorating agent of claim 15, comprising, as an active ingredient, one or more compounds selected from the group consisting of glutamic acid derivatives represented by the formula (1a) and the salts thereof.

17. The rough skin inhibiting or ameliorating agent of claim 16, comprising, as an active ingredient, one or more compounds selected from the group consisting of N-carbamoylglutamic acid, N-benzyloxycarbonylglutamic acid, N-methylglutamic acid, and the salts thereof.

18. The rough skin inhibiting or ameliorating agent of claim 15, comprising, as an active ingredient, one or more compounds selected from the group consisting of glutamic acid derivatives represented by the formula (2) and the salts thereof.

19. The rough skin inhibiting or ameliorating agent of claim 18, wherein Z is a methyl group or a phenyl group.

20. The rough skin inhibiting or ameliorating agent of claim 18 or 19, wherein Y is a hydroxyl group or NR₃R₄.

21. The rough skin inhibiting or ameliorating agent of any of claims 18 to 20, wherein Y is the same as NR₁R₂.

22. The rough skin inhibiting or ameliorating agent of any of claims 18 to 21, wherein at least one of R₁ and R₂, and/or at least one of R₃ and R₄, each independently represents a hydrogen atom, an alkyl group having 1 to 3 carbon atoms, a cycloalkyl group having 3 to 7 carbon atoms, or a benzyl group.

23. The rough skin inhibiting or ameliorating agent of any of claims 18 to 22, wherein at least one of R₁ and R₂, and/or at least one of R₃ and R₄, each independently represents a hydrogen atom, a methyl group, or an ethyl group.

24. The rough skin inhibiting or ameliorating agent of any of claims 18 to 21, wherein R₁ and R₂, and/or R₃ and R₄, independently form the heterocycle having 6 carbon atoms or less in total together with the nitrogen atom to which they are bonded.

25. The rough skin inhibiting or ameliorating agent of claim 18, being one or more compounds selected from N-acetyl-N'-methylglutamic acid-1-amide, N-acetyl-N'-ethylglutamic acid-1-amide, N-acetyl-N'-n-propylglutamic acid-1-amide, N-acetyl-N'-benzylglutamic acid-1-amide, N-acetyl-N'-cyclohexylglutamic acid-1-amide, N-acetyl-N'-cyclopentylglutamic acid-1-amide, N-acetylglutamic acid 1-pyrrolidine amide, N-acetylglutamic acid 1-piperidine amide, N-acetylglutamic acid 1-morpholine amide, N-benzoyl-N'-methylglutamic acid-1-amide, N-benzoyl-N'-ethylglutamic acid-1-amide, N-benzoyl-N'-n-propylglutamic acid-1-amide, N-benzoylglutamic acid 1-morpholine amide, N-acetyl-N',N"-dimethylglutamic acid-1,5-diamide, N-acetyl-N',N"-diethylglutamic acid-1,5-diamide, N-acetyl-N,N',N",N"-tetramethylglutamic acid-1,5-diamide, N-acetyl-N',N',N",N"-tetraethylglutamic acid-1,5-diamide, N-acetylglutamic acid bis-1,5-morpholine amide, N-acetylglutamic acid bis-1,5-piperidine amide, and the salts thereof.

26. An external composition for skin, comprising the rough skin inhibiting or ameliorating agent of any of claims 15 to 25.

27. An external composition for skin, comprising one or more compounds selected from the group consisting of glutamic acid derivatives represented by the formula (1a) or (2) and the salts thereof.
